# EUROPEAN PATENT APPLICATION

(11) **EP 4 826 430 A2**
(43) Date of publication of application: **21.10.2026**
(21) Application number: 26184323.9
(22) Date of filing: 22.06.2021
(51) Int. Cl.: G02B 21/16

(54) **INFORMATION PROCESSING DEVICE, INFORMATION PROCESSING METHOD, PROGRAM, MICROSCOPE SYSTEM, AND ANALYSIS SYSTEM**

(30) Priority: 30.06.2020 JP 2020113405
(62) Document number(s) of the earlier application(s) in accordance with Art. 76 EPC: 21831481.3 / 4 174 554
(71) Applicant: Sony Group Corporation, Tokyo 108-0075 (JP)
(72) Inventor: GOTO, Ai, Tokyo, 108-0075 (JP); NAKAGAWA, Kazuhiro, Tokyo, 108-0075 (JP); NAKAMURA, Tomohiko, Tokyo, 108-0075 (JP); IKEDA, Kenji, Tokyo, 108-0075 (JP)
(74) Representative: D Young & Co LLP

(57) **Abstract**

To improve analysis accuracy in fluorescence microscopy. An information processing device according to an embodiment includes: a separation unit (132) that separates a fluorescence image obtained by observing a specimen labeled with one or more fluorescent dyes into a fluorescence component image containing one or more fluorescence components and an autofluorescence component image containing one or more autofluorescence components; a generation unit (133) that generates an autofluorescence component correction image using a reference spectrum of each of one or more autofluorescent substances included in the specimen and using the autofluorescence component image; and a processing unit (130) that processes the fluorescence component image based on the autofluorescence component correction image.

## Description

### Field

The present disclosure relates to an information processing device, an information processing method, a program, a microscope system, and an analysis system.

### Background

In recent years, along with the development of cancer immunotherapy and the like, there is an increasing need to use a plurality of markers to detect and evaluate molecules that are phenotypes of immune cells classified into a large number of subsets. A multicolor imaging analysis method using a fluorescent dye as a marker is considered to be an effective means of deriving a relationship between localization and function.

### Citation List

### Patent Literature

Patent Literature 1: US Pat. No. 8200013

### Summary

### Technical Problem

In general, when fluorescence microscopy is performed on a stained tissue section stained using a fluorescent dye antibody, the accuracy of measurement of fluorescence intensity representing the amount of antibody might be affected due to autofluorescence or the like. Even when an autofluorescence component is removed from a captured image of the stained tissue section by color separation using the spectrum, it has not been possible to completely eliminate the autofluorescence component remaining in the stained fluorescence image obtained by the color separation.

The autofluorescence component remaining in the stained fluorescence image in this manner causes a problem such as hindering quantification of the amount of dye antibody distributed in an analysis target region in the stained fluorescence image, which has hindered improvement of analysis accuracy in fluorescence microscopy.

The present disclosure has been made in view of the above circumstances, and proposes an information processing device, an information processing method, a program, a microscope system, and an analysis system capable of improving analysis accuracy in the fluorescence microscopy.

### Solution to Problem

An information processing device according to an embodiment of the present disclosure including: a separation unit that separates a fluorescence image obtained by observing a specimen labeled with one or more fluorescent dyes into a fluorescence component image containing one or more fluorescence components and an autofluorescence component image containing one or more autofluorescence components; a generation unit that generates an autofluorescence component correction image using a reference spectrum of each of one or more autofluorescent substances included in the specimen and using the autofluorescence component image; and a processing unit that processes the fluorescence component image based on the autofluorescence component correction image.

### Brief Description of Drawings

FIG. 1 is a block diagram illustrating a configuration example of an information processing system according to a first embodiment.
FIG. 2 is a diagram illustrating a specific example of a fluorescence spectrum acquired by a fluorescence signal acquisition unit.
FIG. 3 is a diagram illustrating an outline of non-negative matrix factorization.
FIG. 4 is a diagram illustrating an outline of clustering.
FIG. 5 is a block diagram illustrating a configuration example of a microscope system in a case where the information processing system according to the first embodiment is implemented as a microscope system.
FIG. 6 is a flowchart illustrating a basic operation example of the information processing system according to the first embodiment.
FIG. 7 is a flowchart illustrating an operation example when generating a pseudo autofluorescence component image according to the first embodiment.
FIG. 8 is a diagram illustrating an example of an autofluorescence component image generated in Step S103 of FIG. 6.
FIG. 9 is a diagram illustrating an example of an autofluorescence reference spectrum included in specimen information acquired in Step S102 in FIG. 6.
FIG. 10 is a diagram illustrating Step S112 in FIG. 7.
FIG. 11 is a diagram illustrating Step S114 in FIG. 7.
FIG. 12 is a flowchart illustrating extraction processing and analysis of an analysis target region according to a comparative example of the first embodiment.
FIG. 13 is a schematic diagram illustrating extraction processing and analysis of an analysis target region according to the comparative example illustrated in FIG. 12.
FIG. 14 is a flowchart illustrating extraction processing and analysis of an analysis target region according to the first embodiment.
FIG. 15 is a schematic diagram illustrating extraction processing and analysis of an analysis target region according to the present embodiment illustrated in FIG. 14.
FIG. 16 is a flowchart illustrating extraction processing and analysis of an analysis target region according to a second embodiment.
FIG. 17 is a schematic diagram illustrating extraction processing and analysis of an analysis target region according to the present embodiment illustrated in FIG. 16.
FIG. 18 is a flowchart illustrating generation processing and analysis of a spectral intensity ratio image according to a third embodiment.
FIG. 19 is a diagram illustrating a first generation method according to the third embodiment.
FIG. 20 is a diagram illustrating a second generation method according to the third embodiment.
FIG. 21 is a flowchart illustrating fluorescence component image generation processing using machine learning according to a fourth embodiment.
FIG. 22 is a schematic diagram illustrating fluorescence component image generation processing using machine learning according to the present embodiment illustrated in FIG. 21.
FIG. 23 is a diagram illustrating an example of a measurement system of the information processing system according to the embodiment.
FIG. 24 is a diagram illustrating a method of calculating the number of fluorescent molecules (or the number of antibodies) in one pixel according to the embodiment.
FIG. 25 is a block diagram illustrating a hardware configuration example of an information processing device according to each embodiment and modification.

### Description of Embodiments

A preferred embodiment of the present disclosure will be described in detail hereinbelow with reference to the accompanying drawings. Note that redundant descriptions will be omitted from the present specification and the drawings by assigning the same reference signs to components having substantially the same functional configuration.

Note that the description will be provided in the following order.
0. Introduction
1. First embodiment
1.1. Configuration examples
1.2. Example of application to microscope system
1.3. Least squares method
1.4. Non-negative Matrix Factorization (NMF)
1.5. Characteristic configuration of first embodiment
1.5.1. Basic operation example
1.5.2. Example of autofluorescence component correction image generation flow
1.6. Extraction and analysis of analysis target region
1.7. Action and effects
2. Second embodiment
2.1. Extraction and analysis of analysis target region
2.2. Action and effects
3. Third embodiment
3.1. Generation and analysis of spectral intensity ratio image
3.2. Method of generating spectral intensity image
3.2.1 First generation method
3.2.2 Second generation method
3.3. Action and effects
4. Fourth embodiment
4.1. Generation of fluorescence component image using machine learning
4.2. Action and effects
5. Configuration example of measurement system
6. Method of calculating number of fluorescent molecules (or number of antibodies)
7. Hardware configuration example

### <0. Introduction>

In recent years, along with the development of cancer immunotherapy and the like, there is an increasing need to use a plurality of markers to detect and evaluate molecules that are phenotypes of immune cells classified into a large number of subsets. A multicolor imaging analysis method using a fluorescent dye as a marker is considered to be an effective means of deriving a relationship between localization and function, ad is adopted in the following embodiments.

The following embodiment relates to image processing after color separation processing is performed on a multi-channel image (number of pixels × wavelength channel (CH)) obtained by imaging a pathological specimen section immunohistochemically stained with excitation light of a plurality of wavelengths by using an imaging device that performs fluorescence separation.

Note that the multi-channel image in the present disclosure can include various images having a data cube structure, including image data of a plurality of wavelength channels (not excluding a single wavelength channel), such as a stained specimen image, a fluorescence component image, a fluorescence component correction image, an autofluorescence component image, and an autofluorescence component correction image, which are to be described below. Accordingly, each of the fluorescence component image, the fluorescence component correction image, the autofluorescence component image, and the autofluorescence component correction image is not limited to the image data of the fluorescence component or the autofluorescence component of a single wavelength channel, and may be a spectral image including the fluorescence components or the autofluorescence components of a plurality of wavelength channels. In addition, the fluorescence component and the autofluorescence component in the present description mean wavelength components of fluorescence or autofluorescence in a multi-channel image obtained by imaging with an imaging device.

As described above, when fluorescence microscopy is performed on a stained tissue section stained using a fluorescent dye antibody, the accuracy of measurement of fluorescence intensity representing the amount of antibody might be affected due to autofluorescence or the like. In addition, even with execution of processing (hereinafter, also referred to as a color separation processing) of removing spectral information derived from an autofluorescent substance (hereinafter, referred to as an autofluorescence component or an autofluorescence spectrum) by color separation using a spectrum from a captured image (hereinafter, referred to as stained specimen image) of a stained tissue section (hereinafter, also referred to as a stained section), it has not been possible to completely eliminate the autofluorescence component included in the stained specimen image (referred to as a fluorescence component image) after the color separation processing. This limitation has been a factor that causes problems such as hindrance in quantification of the amount of dye antibodies distributed in the analysis target region in the stained specimen image.

In view of this situation, in order to solve these problems, the embodiments below proposes a method having characteristics described below in relation to a process of generating a pseudo autofluorescence component image (autofluorescence component correction image) from a color separation image (spectral image of each fluorescence component and/or each autofluorescence component, i.e. fluorescence component image and/or autofluorescence component image) of a stained section, and in relation to image processing using the generated image.

As a first characteristic, the following embodiment includes a process of generating an image (autofluorescence component correction image) in which the spectral intensity of autofluorescence is calculated, from a fluorescence component image (also referred to as an antibody number image) including an autofluorescence component extracted from a stained specimen image by color separation processing and spectral information (fluorescence component, also referred to as fluorescence spectrum) derived from a fluorescent substance obtained by color separation processing. By using such an autofluorescence component correction image, it is possible to achieve effects such as reduction of labor of separately acquiring an autofluorescence component image acquired by imaging an unstained section, and improvement of quantitativity by using spectrum information associated with position information.

As a second characteristic, luminance threshold processing is performed on the stained specimen image using the autofluorescence component correction image generated from the stained specimen image. This makes it possible to extract a region having a specific signal of a fluorescent substance (also referred to as a stained fluorescent dye) distinguished from autofluorescence.

As a third characteristic, there is a process of performing, similarly for the fluorescent substance, generation of an image (fluorescence component correction image) with calculated spectral intensity of fluorescence from the fluorescence component derived from the fluorescent substance and the fluorescence component image obtained by the color separation processing, and calculation of a relative ratio between the spectral intensity derived from the fluorescent substance and the spectral intensity derived from the autofluorescent substance. This also makes it possible to evaluate color separation accuracy and a fluorescent dye antibody panel designed.

Patent Literature 1 described above proposes a method of setting a threshold based on a difference in pixel luminance values and extracting the target. However, since it is not clear that there is a correlation between the difference in luminance values and the presence or absence of expression of molecules, there is a possibility that extraction of an object by such a method is not appropriate. In this regard, the embodiments below extract the target region using the autofluorescence component correction image confirmed to have a correlation between the difference in luminance values and the presence or absence of expression of the molecule, making it possible to appropriately extract the target region.

On the other hand, a technology referred to as in silico labeling technology (Eric M. Christiansen et al., "In Silico Labeling: Predicting Fluorescent Labels in Unlabeled Images", Cell, 173, 792-803, April 19, 2018) has convinced that it is possible to predict a target cell region from a transmission image (autofluorescence component image) by utilizing deep learning so as to reproduce a fluorescence-labeled image with high accuracy. This technology, however, has a disadvantage that, while it is necessary to prepare an actual fluorescence-labeled image as training data together as an input image, it is not possible to prepare two input images (an autofluorescence component image and a fluorescence-labeled image) having the same visual field, and thus serial sections have to be used, leading to occurrence of a spatial difference between the two input images. In this regard, the embodiments below generate a pseudo autofluorescence component image from a stained specimen image acquired by imaging a stained section, making it possible to discuss quantitativity using data of the same population.

Furthermore, it is conceivable to perform region designation using serial sections of an identical tissue block, but even in the case of using serial sections, images obtained by capturing a region shifted by about several µm in the optical axis direction are to be used, leading to a possibility that the unstained section and the stained section are affected by a spatial difference in terms of the distribution form of cells and autofluorescent substances, signal strength, and the like. In this regard, the embodiments below generate an autofluorescence component correction image based on spectral information of the autofluorescent substance extracted from the stained section itself, it is possible to quantify the stained specimen image under the condition that the variations of the autofluorescent substance and the like are spatially the same.

### <1. First embodiment>

First, a first embodiment according to the present disclosure will be described.

### <<1.1. Configuration examples>>

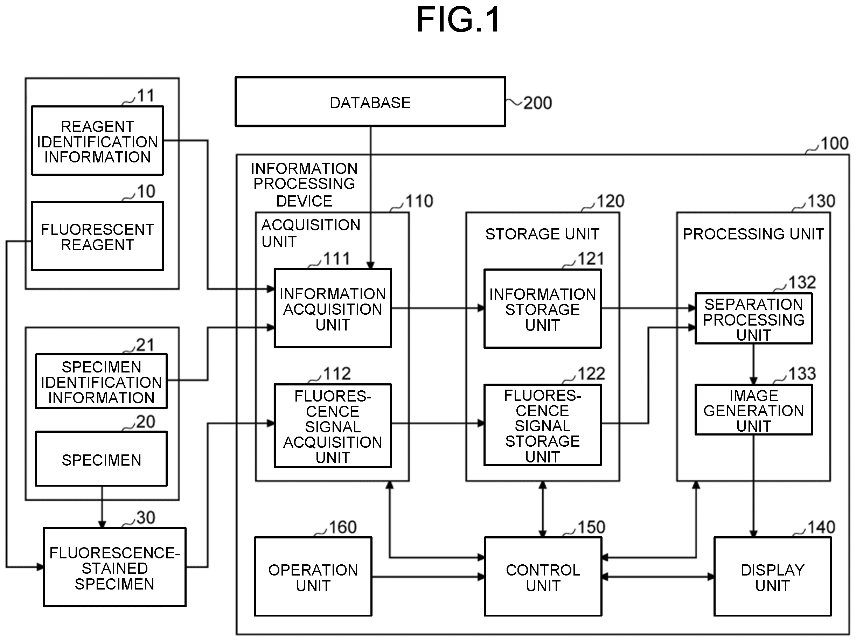

First, an example of a configuration of an information processing system according to the present embodiment will be described with reference to FIG. 1. As illustrated in FIG. 1, the information processing system according to the present embodiment includes an information processing device 100 and a database 200, and there are a fluorescent reagent 10, a specimen 20, and a fluorescence-stained specimen 30, as inputs to the information processing system.

### (Fluorescent reagent 10)

The fluorescent reagent 10 is a chemical used for staining the specimen 20. Example of the fluorescent reagent 10 include a fluorescent antibody (primary antibodies used for direct labeling or secondary antibodies used for indirect labeling), a fluorescent probe, and a nuclear staining reagent, although the type of the fluorescent reagent 10 is not limited to these. In addition, the fluorescent reagent 10 is managed with identification information (hereinafter referred to as "reagent identification information 11") by which the fluorescent reagent 10 (or the production lot of the fluorescent reagent 10) is identifiable. The reagent identification information 11 is bar code information (one-dimensional barcode information, two-dimensional barcode information, or the like), for example, but is not limited thereto. Even in the case of the same product, the properties of the fluorescent reagent 10 are different for each production lot according to the conditions such as the production method and the state of the cell from which the antibody is acquired. For example, the fluorescent reagent 10 has a difference in a wavelength spectrum of fluorescence (fluorescence spectrum), a quantum yield, a fluorescent labeling rate, and the like, for each production lot. Therefore, in the information processing system according to the present embodiment, the fluorescent reagent 10 is managed for each production lot with the reagent identification information 11 attached. With this management, the information processing device 100 can perform fluorescence separation in consideration of a slight difference in properties that appears for each production lot.

### (Specimen 20)

The specimen 20 is prepared for the purpose of pathological diagnosis or the like from a specimen or a tissue sample collected from a human body. The specimen 20 may be a tissue section, a cell, or a fine particle, and there is no particular limitation regarding details of the specimen 20, such as the type of tissue (for example, organ or the like) used, the type of disease to be targeted, attributes of the subject (for example, age, sex, blood type, race, and the like), or the lifestyle of the subject (for example, dietary habits, exercise habits, and smoking habits). Incidentally, a tissue section can include, for example, a section before staining of a tissue section to be stained (hereinafter, it is also simply referred to as a section), a section adjacent to the stained section, a section different from the stained section in a same block (sampled from the same location as the stained section), a section in a different block in a same tissue (sampled from a different place from the stained section), and a section collected from a different patient. In addition, the specimen 20 is managed with identification information (hereinafter, referred to as "specimen identification information 21") by which each specimen 20 is identifiable. Similarly to the reagent identification information 11, the specimen identification information 21 includes but not limited to barcode information (one-dimensional barcode information, two-dimensional barcode information, or the like, for example. The specimen 20 varies in nature depending on the type of organization used, the type of the target disease, the attribute of the subject, the lifestyle of the subject, or the like. For example, the specimen 20 has a different measurement channel, a wavelength spectrum of autofluorescence (autofluorescence spectrum), or the like, according to the type of tissue to be used, or the like. Therefore, in the information processing system according to the present embodiment, the specimens 20 are individually managed with the specimen identification information 21 attached. With this management, the information processing device 100 can perform fluorescence separation in consideration of a slight difference in properties that appears for each specimen 20.

### (Fluorescence-stained specimen 30)

The fluorescence-stained specimen 30 is prepared by staining the specimen 20 with the fluorescent reagent 10. In the present embodiment, the fluorescence-stained specimen 30 is assumed to be obtained by staining the specimen 20 with one or more fluorescent reagents 10, through the number of fluorescent reagents 10 used for staining is not particularly limited. The staining method is determined by a combination of the specimen 20 and the fluorescent reagent 10, or example, and is not particularly limited.

### (Information processing device 100)

As illustrated in FIG. 1, the information processing device 100 includes an acquisition unit 110, a storage unit 120, a processing unit 130, a display unit 140, a control unit 150, and an operation unit 160. The information processing device 100, which can be implemented as a fluorescence microscope, for example, is not necessarily limited thereto, and may be implemented as various devices. For example, the information processing device 100 may be a personal computer (PC) or the like.

### (Acquisition Unit 110)

The acquisition unit 110 is configured to acquire information used for various types of processing of the information processing device 100. As illustrated in FIG. 1, the acquisition unit 110 includes an information acquisition unit 111 and a fluorescence signal acquisition unit 112.

### (Information acquisition unit 111)

The information acquisition unit 111 is configured to acquire information related to the fluorescent reagent 10 (hereinafter, referred to as "reagent information") and information related to the specimen 20 (hereinafter, referred to as "specimen information"). More specifically, the information acquisition unit 111 acquires the reagent identification information 11 attached to the fluorescent reagent 10 used for generating the fluorescence-stained specimen 30 and the specimen identification information 21 attached to the specimen 20. For example, the information acquisition unit 111 acquires the reagent identification information 11 and the specimen identification information 21 using a barcode reader or the like. The information acquisition unit 111 acquires the reagent information based on the reagent identification information 11 and acquires the specimen information from the database 200 based on the specimen identification information 21. The information acquisition unit 111 stores the acquired information in an information storage unit 121 described below.

Here, the present embodiment assumes that the specimen information includes information regarding the autofluorescence spectrum (hereinafter, also referred to as autofluorescence reference spectrum) of one or more autofluorescent substances in the specimen 20, and the reagent information includes information regarding the fluorescence spectrum (hereinafter, also referred to as a fluorescence reference spectrum) of the fluorescent substance in the fluorescence-stained specimen 30. The autofluorescence reference spectrum and the fluorescence reference spectrum are also individually or collectively referred to as "reference spectrum".

### (Fluorescence signal acquisition unit 112)

The fluorescence signal acquisition unit 112 is configured to acquire a plurality of fluorescence signals each corresponding to a plurality of excitation light beams having different wavelengths when the fluorescence-stained specimen 30 (created by staining the specimen 20 with the fluorescent reagent 10) is irradiated with the plurality of excitation light beams. More specifically, the fluorescence signal acquisition unit 112 receives light and outputs a detection signal corresponding to the amount of received light, thereby acquiring a data cube (hereinafter, referred to as stained specimen image) constituted with the fluorescence spectrum of the fluorescence-stained specimen 30 based on the detection signal. Here, the types of the excitation light (including the excitation wavelength, the intensity, and the like) are determined based on reagent information and the like (in other words, information related to the fluorescent reagent 10, and the like). Note that the fluorescence signal herein is not particularly limited as long as it is a signal derived from fluorescence, and the fluorescence spectrum is merely an example the signal. In the present description, a case where the fluorescence signal is a fluorescence spectrum will be exemplified.

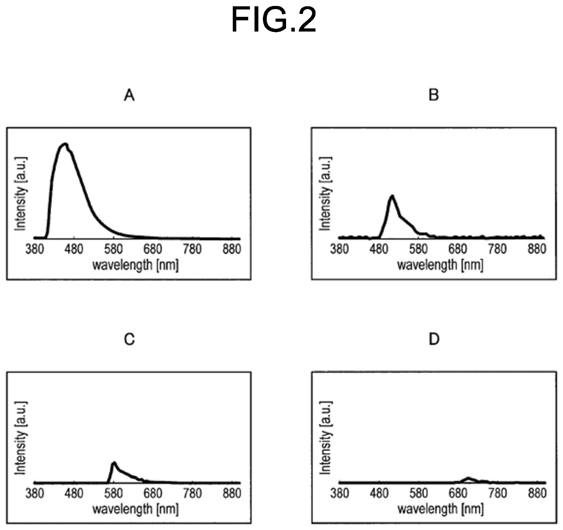

A to D of FIG. 2 are specific examples of the fluorescence spectrum acquired by the fluorescence signal acquisition unit 112. Drawings A to D of FIG. 2 each illustrate a specific example of fluorescence spectrum when the fluorescence-stained specimen 30 contains four types of fluorescent substance of DAPI, CK/AF 488, PgR/AF 594, and ER/AF647, and the specimen is irradiated with excitation light beams having excitation wavelengths of 392[nm] (A of FIG. 2), 470[nm] (B of FIG. 2), 549[nm] (C of FIG. 2), and 628[nm] (D of FIG. 2). Note that the fluorescence wavelength is shifted to a longer wavelength side than the excitation wavelength due to the emission of energy for fluorescence emission (Stokes shift). Also note that the fluorescent substance contained in the fluorescence-stained specimen 30 and the excitation wavelength of the excitation light to be emitted are not limited to the above. The fluorescence signal acquisition unit 112 stores a stained specimen image having the acquired fluorescence spectrum in a fluorescence signal storage unit 122 described below.

### (Storage unit 120)

The storage unit 120 is configured to store information used for various types of processing of the information processing device 100 or information output by the various types of processing. As illustrated in FIG. 1, the storage unit 120 includes the information storage unit 121 and the fluorescence signal storage unit 122.

### (Information storage unit 121)

The information storage unit 121 is configured to store the reagent information and the specimen information acquired by the information acquisition unit 111.

### (Fluorescence signal storage unit 122)

The fluorescence signal storage unit 122 is configured to store the fluorescence signal of the fluorescence-stained specimen 30 acquired by the fluorescence signal acquisition unit 112.

### (Processing unit 130)

The processing unit 130 is configured to perform various types of processing including color separation processing. As illustrated in FIG. 1, the processing unit 130 includes a separation processing unit 132 and an image generation unit 133.

### (Separation processing unit 132)

The separation processing unit 132 is configured to separate the stained specimen image into a fluorescence spectrum for each fluorescent substance, and extracts an autofluorescence spectrum from the input stained specimen image and generates an autofluorescence component correction image using the extracted autofluorescence spectrum as described below (generation unit). The separation processing unit 132 then executes color separation processing of the stained specimen image using the generated autofluorescence component correction image (separation unit). The separation processing unit 132 can function as a generation unit, a separation unit, a correction unit, and an image generation unit in the claims.

The color separation processing may use a least squares method (LSM), a weighted least squares method (WLSM), or the like. In addition, extraction of the autofluorescence spectrum and/or the fluorescence spectrum may use non-negative matrix factorization (NMF), singular value decomposition (SVD), principal component analysis (PCA), or the like.

### (Operation unit 160)

The operation unit 160 is configured to receive an operation input from a technician. More specifically, the operation unit 160 includes various input means such as a keyboard, a mouse, a button, a touch panel, or a microphone, and the technician can perform various inputs to the information processing device 100 by operating these input means. Information regarding the operation input performed via the operation unit 160 is provided to the control unit 150.

### (Database 200)

The database 200 is a device that manages reagent information, specimen information, and the like. More specifically, the database 200 manages the reagent identification information 11 in association with the reagent information, and the specimen identification information 21 in association with the specimen information. With this management, the information acquisition unit 111 can acquire the reagent information based on the reagent identification information 11 of the fluorescent reagent 10 and can acquire the specimen information from the database 200 based on the specimen identification information 21 of the specimen 20.

The reagent information managed by the database 200 is assumed to be (but not limited to) information including a measurement channel unique to the fluorescent substance of the fluorescent reagent 10 and a fluorescence reference spectrum. The "measurement channel" is a concept indicating a fluorescent substance contained in the fluorescent reagent 10. Since the number of fluorescent substances varies depending on the fluorescent reagent 10, the measurement channel is managed in association with each fluorescent reagent 10 as reagent information. As described above, the fluorescence reference spectrum included in the reagent information is the fluorescence spectrum of each fluorescent substance included in the measurement channel.

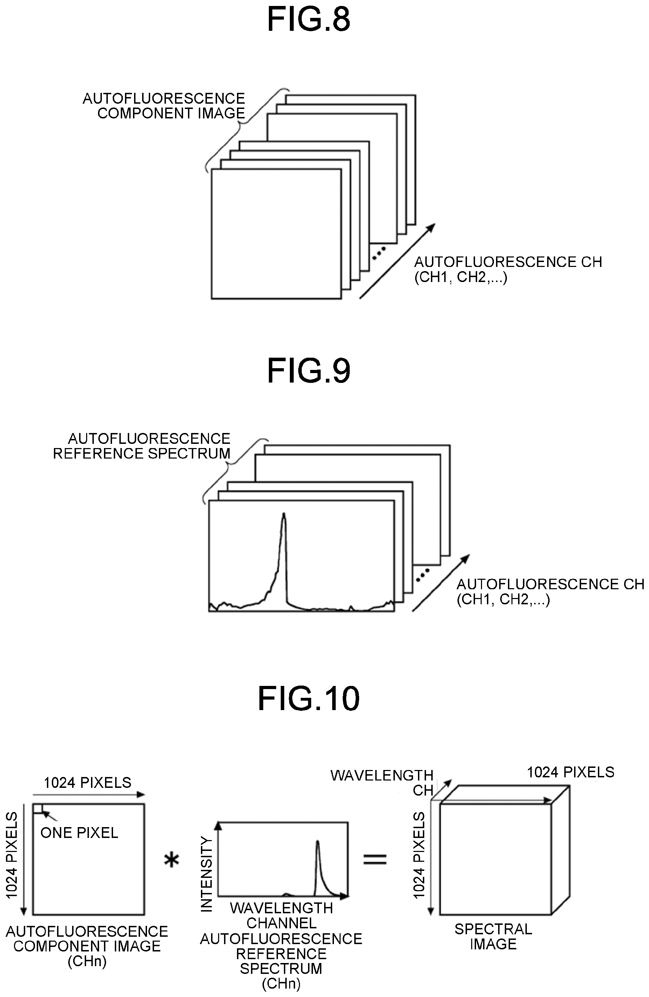

In addition, the specimen information managed by the database 200 is assumed to be (but not limited to) information including a measurement channel unique to the autofluorescent substance of the specimen 20 and an autofluorescence reference spectrum. The "measurement channel" is a concept indicating an autofluorescent substance contained in the specimen 20, and a concept indicating, in the example of FIG. 8, Hemoglobin, Archidonic Acid, Catalase, Collagen, FAD, NADPH, and ProLongDiamond. Since the number of autofluorescent substances varies depending on the specimen 20, the measurement channel is managed in association with each specimen 20 as specimen information. As described above, the autofluorescence reference spectrum included in the specimen information is the autofluorescence spectrum of each autofluorescent substance included in the measurement channel. Note that the information managed by the database 200 is not necessarily limited to the above.

The configuration example of the information processing system according to the present embodiment has been described as above. The above configuration described with reference to FIG. 1 is merely an example, and the configuration of the information processing system according to the present embodiment is not limited to such an example. For example, the information processing device 100 do not necessarily have to include all of the components illustrated in FIG. 1, or may include a component not illustrated in FIG. 1.

Here, the information processing system according to the present embodiment may include: an imaging device (including a scanner or the like, for example) that acquires a fluorescence spectrum; and an information processing device that performs processing using the fluorescence spectrum. In this case, the fluorescence signal acquisition unit 112 illustrated in FIG. 1 can be implemented by the imaging device, and other configurations can be implemented by the information processing device. Furthermore, the information processing system according to the present embodiment may include: an imaging device that acquires a fluorescence spectrum; and software used for processing using the fluorescence spectrum. In other words, the physical configuration (for example, memory, a processor, or the like) for storing and executing the software need not be provided in the information processing system. In this case, the fluorescence signal acquisition unit 112 illustrated in FIG. 1 can be implemented by the imaging device, and other configurations can be implemented by the information processing device on which the software is executed. The software is provided to the information processing device via a network (for example, from a website, a cloud server, or the like) or provided to the information processing device via a certain storage medium (for example, a disk or the like). Furthermore, the information processing device on which the software is executed may be various servers (for example, a cloud server or the like), a general-purpose computer, a PC, a tablet PC, or the like. Note that the method by which the software is provided to the information processing device and the type of the information processing device are not limited to the above. Furthermore, it should be noted that the configuration of the information processing system according to the present embodiment is not necessarily limited to the above, and a configuration conceivable to a person skilled in the art can be applied based on the technical level at the time of use.

### (1.2. Example of application to microscope system)

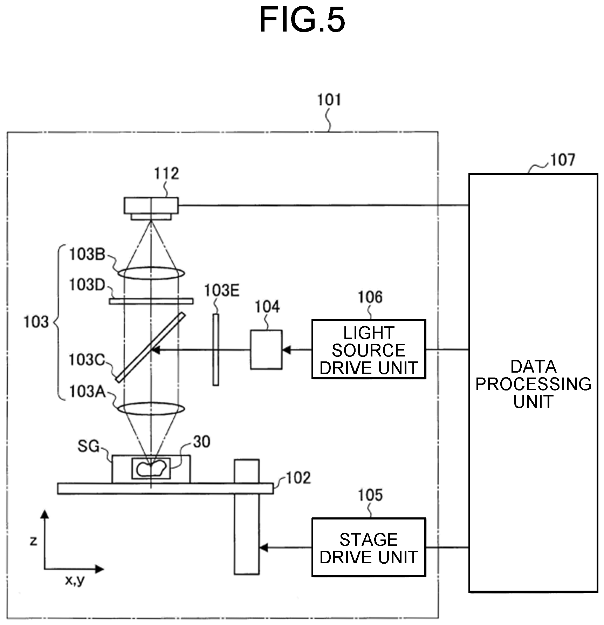

The information processing system described above may be implemented as a microscope system, for example. Therefore, next, a configuration example of a microscope system in a case where the information processing system according to the present embodiment is implemented as a microscope system will be described with reference to FIG. 5.

As illustrated in FIG. 5, the microscope system according to the present embodiment includes a microscope 101 and a data processing unit 107.

The microscope 101 includes a stage 102, an optical system 103, a light source 104, a stage drive unit 105, a light source drive unit 106, and a fluorescence signal acquisition unit 112.

Having a placement surface on which the fluorescence-stained specimen 30 can be placed, the stage 102 is movable in a direction parallel to the placement surface (x-y plane direction) and a direction perpendicular to the placement surface (z-axis direction) by drive of the stage drive unit 105. The fluorescence-stained specimen 30 has a thickness of, for example, several µm to several tens µm in the Z direction, and is fixed by a predetermined fixing method while being sandwiched between a glass slide SG and a cover slip (not illustrated).

The optical system 103 is disposed above the stage 102. The optical system 103 includes an objective lens 103A, an imaging lens 103B, a dichroic mirror 103C, an emission filter 103D, and an excitation filter 103E. The light source 104 is, for example, a light bulb such as a mercury lamp, a light emitting diode (LED), or the like, and emits excitation light to a fluorescent label attached to the fluorescence-stained specimen 30 by the drive of the light source drive unit 106.

In acquisition of a fluorescence image of the fluorescence-stained specimen 30, the excitation filter 103E transmits only a light beam having an excitation wavelength that excites the fluorescent dye among the light beam emitted from the light source 104, thereby generating excitation light. The dichroic mirror 103C reflects the excitation light incident after being transmitted through the excitation filter, and guides the reflected excitation light to the objective lens 103A. The objective lens 103A condenses the excitation light on the fluorescence-stained specimen 30. The objective lens 103A and the imaging lens 103B magnify the image of the fluorescence-stained specimen 30 to a predetermined magnification, and form a magnified image on an imaging surface of the fluorescence signal acquisition unit 112.

When the fluorescence-stained specimen 30 is irradiated with excitation light, the stain bound to each tissue of the fluorescence-stained specimen 30 emits fluorescence. This fluorescence is transmitted through the dichroic mirror 103C via the objective lens 103A and reaches the imaging lens 103B via the emission filter 103D. The emission filter 103D absorbs the light magnified by the objective lens 103A and transmitted through the excitation filter 103E, and transmits only a part of the color-emission light. As described above, the image of the color-emission light in which the external light is lost is magnified by the imaging lens 103B and formed on the fluorescence signal acquisition unit 112.

The data processing unit 107 is configured to drive the light source 104, acquire a fluorescence image of the fluorescence-stained specimen 30 using the fluorescence signal acquisition unit 112, and perform various types of processing using the fluorescence image acquired. More specifically, the data processing unit 107 can function as a part or all of the configuration of the information acquisition unit 111, the storage unit 120, the processing unit 130, the display unit 140, the control unit 150, the operation unit 160, or the database 200 of the information processing device 100 described with reference to FIG. 1. For example, by functioning as the control unit 150 of the information processing device 100, the data processing unit 107 controls the drive of the stage drive unit 105 and the light source drive unit 106 as well as controlling the acquisition of the spectrum by the fluorescence signal acquisition unit 112. Furthermore, by functioning as the processing unit 130 of the information processing device 100, the data processing unit 107 generates a fluorescence spectrum, separate a fluorescence spectrum for each fluorescent substance, and generates image information based on a result of the separation.

The configuration example of a microscope system in a case where the information processing system according to the present embodiment is implemented as a microscope system has been described as above. The above configuration described with reference to FIG. 5 is merely an example, and the configuration of the microscope system according to the present embodiment is not limited to such an example. For example, the microscope system do not necessarily have to include all of the components illustrated in FIG. 5, or may include a component not illustrated in FIG. 5.

### (1.3. Least squares method)

Here, the least squares method used in the color separation processing by the separation processing unit 132 will be described. The least squares method is a method of calculating a color mixing ratio by fitting a reference spectrum to a fluorescence spectrum, which is a pixel value of each pixel in the input stained specimen image. The color mixing ratio is an index indicating the degree of mixing of individual substances. The following formula (1) is an expression representing a residual obtained by subtracting the mixture of the reference spectrum (St), the fluorescence reference spectrum, and the autofluorescence reference spectrum at a color mixture ratio a, from the fluorescence spectrum (signal). Note that "Signal (1 × the number of channels)" in Formula (1) indicates that the fluorescence spectrum (Signal) exists as many as the number of channels of the wavelength. For example, Signal is a matrix representing one or more fluorescence spectra. "St (number of substances × number of channels)" indicates that the reference spectrum exists as many as the number of channels of the wavelength for individual substances (fluorescent substance and autofluorescent substance). For example, St is a matrix representing one or more reference spectra. In addition, "a (1 × the number of substances)" indicates that the color mixing ratio a is provided for individual substances (fluorescent substance and autofluorescent substance). For example, a is a matrix representing a color mixing ratio of each reference spectrum in the fluorescence spectrum.$\begin{matrix}Signal \left(1\times Number of channels\right) \\ - a \left(1\times Number of substances\right) * {S}_{t} \left(\begin{matrix}Number of substances \\ \times Number of channels\end{matrix}\right)\end{matrix}$ (1)

Subsequently, the separation processing unit 132 calculates the color mixing ratio a of each substance having the minimum sum of squares of the Formula (1) indicating the residual. The sum of squares of the residuals is minimized in a case where the result of partial differentiation with respect to the color mixing ratio a is 0 in Formula (1) indicating the residual. Accordingly, by solving the following Formula (2), the separation processing unit 132 calculates the color mixing ratio a of each substance which achieves the minimum sum of squares of the residuals. Note that "St'" in Formula (2) indicates a transposed matrix of the reference spectrum St. Furthermore, "inv(St*St')" represents an inverse matrix of St*St'.$\begin{matrix}\frac{δ \left(Signal-a*St\right)}{δ a} = 0 \\ ⇔ 2 \left(Signal-a*St\right) * St ′ = 0 \\ ⇔ \left(Signal-a*St\right) * St ′ = 0 \\ ⇔ Signal * St ′ - a * \left(St*St′\right) = 0 \\ ⇔ a = Signal * St ′ * inv \left(St*St′\right)\end{matrix}$ (2)

Here, specific examples of each value of the above formula (1) are illustrated in the following Formulas (3) to (5). In the examples of Formulas (3) to (5), the reference spectrum (St) of three substances (the number of substances is three) are mixed at different color mixing ratios a in the fluorescence spectrum (Signal).$St = \left(\begin{matrix}50 & 100 & 60 & 25 & 4 \\ 10 & 20 & 100 & 20 & 8 \\ 0.1 & 11 & 30 & 100 & 50\end{matrix}\right)$ (3) $a = \left(3 2 1\right)$ (4) $Signal = a * St = \left(170.1 351 410 215 78\right)$ (5)

A specific example of the calculation result of the above Formula (2) based on each value of Formulas (3) and (5) is illustrated in the following Formula (6). As indicated in Formula (6), "a=(3 2 1)" (that is, the same value as the above Formula (4)) is correctly calculated as the calculation result.$a = Signal * St ′ * inv \left(St*St′\right) = \left(3 2 1\right)$ (6)

Note that, as described above, the separation processing unit 132 may extract the spectrum for each fluorescent substance from the fluorescence spectrum by performing calculation related to the weighted least squares method instead of the least squares method. In the weighted least squares method, by utilizing the fact that the noise of the fluorescence spectrum (Signal), which is a measured value, has a Poisson distribution, weighting is performed so as to emphasize an error of a low signal level. However, an upper limit value at which weighting is not performed by the weighted least squares method is set as an offset value. The offset value is determined by characteristics of a sensor used for measurement, and in a case where an imaging element is used as a sensor, it is necessary to separately optimize the offset value. When the weighted least squares method is performed, the reference spectrum St in the above Formulas (1) and (2) is replaced with St_ expressed by the following Formula (7). The following Formula (7) operates to calculate St_ by dividing using each element (in other words, element division) performed such that each element (each component) of St represented by the matrix is divided by each corresponding element (each component) in the "Signal + Offset value" represented by a matrix as well.${S}_{t} = \frac{{S}_{t}}{Signal \times Offset value}$ (7)

Here, a specific example of St_ expressed by the above Formula (7) when the Offset value is 1 and the values of the reference spectrum St and the fluorescence spectrum Signal are respectively expressed by the above Formulas (3) and (5) is expressed by Formula (8) below.$\begin{matrix}{S}_{t} = \frac{{S}_{t}}{Signal \times Offset value} \\ = \left(\begin{matrix}0.2922 & 0.2841 & 0.1460 & 0.1157 & 0.0506 \\ 0.0584 & 0.0568 & 0.2433 & 0.0926 & 0.1013 \\ 5.8445 {e}^{- 5} & 0.0313 & 0.0730 & 0.4630 & 0.6329\end{matrix}\right)\end{matrix}$ (8)

A specific example of the calculation result of the color mixing ratio a in this case will be given as the following Formula (9). As indicated in Formula (9), "a=(3 2 1)" is correctly calculated as the calculation result.$a = Signal * St _ ′ * inv \left(St*St_′\right) = \left(3 2 1\right)$ (9)

### (1.4. Non-negative Matrix Factorization (NMF))

Next, non-negative matrix factorization (NMF) used by the separation processing unit 132 to extract an autofluorescence spectrum and/or a fluorescence spectrum will be described. However, the method of extraction is not limited to non-negative matrix factorization (NMF), and singular value decomposition (SVD), principal component analysis (PCA), or the like may be used.

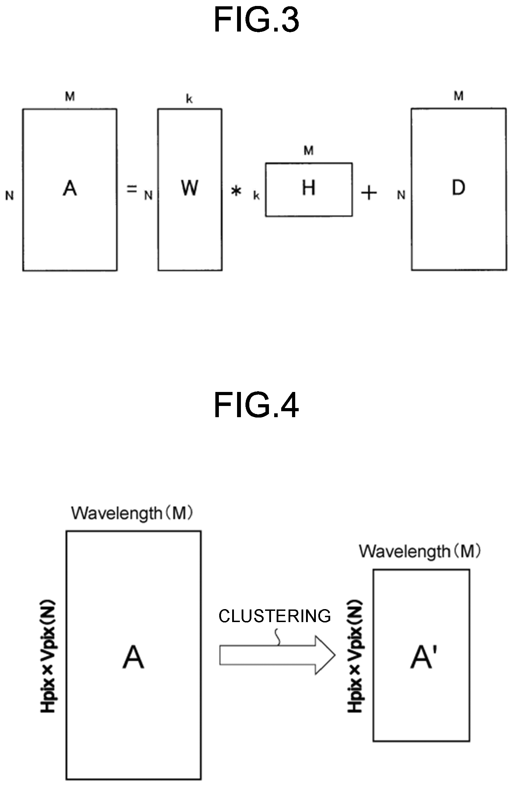

FIG. 3 is a diagram illustrating an outline of NMF. As illustrated in FIG. 3, the NMF decomposes matrix A of non-negative N rows and M columns (N × M) into matrix W of non-negative N rows and k columns (N × k) and a matrix H of non-negative k rows and M columns (k × M). The matrix W and the matrix H are determined so as to minimize a mean square residual D between the matrix A and the product (W*H) of the matrix W and the matrix H. In the present embodiment, matrix A corresponds to the spectrum before the autofluorescence reference spectrum is extracted (N is the number of pixels, and M is the number of wavelength channels), and matrix H corresponds to the extracted autofluorescence reference spectrum (k is the number of autofluorescence reference spectrum (in other words, the number of autofluorescent substances) and M is the number of wavelength channels). Here, the mean square residual D is expressed by the following Formula (10). The "norm (D, 'fro')" refers to the Frobenius norm of the mean square residual D. $D = \frac{norm \left(D,′fro′\right)}{\sqrt{N * M}}$ (10)

Factorization in NMF uses an iterative method starting with random initial values for the matrix W and the matrix H. In the NMF, the value k (the number of autofluorescence reference spectra) is essential. However, the initial values of the matrix W and the matrix H are not essential and can be set as options. When the initial values of the matrix W and the matrix H are set, the solution is constant. On the other hand, in a case where the initial values of the matrix W and the matrix H are not set, these initial values are randomly set, and the solution is not constant.

The specimen 20 has different properties depending on the type of tissue used, the type of the target disease, the attribute of the subject, the lifestyle of the subject, or the like, and has different autofluorescence spectra. Therefore, the information processing device 100 according to the second embodiment can implement color separation processing with higher accuracy by actually measuring the autofluorescence reference spectrum for each specimen 20 as described above.

Note that matrix A, which is an input of the NMF, is a matrix formed with the same number of rows as the number of pixels N (= Hpix × Vpix) of the stained specimen image and the same number of columns as the number of wavelength channels M, as described above. Therefore, when there is a large number of pixels of the stained specimen image or a large number of wavelength channels M, the matrix A will be a very large matrix, increasing the calculation cost of the NMF with prolonged processing time.

In such a case, for example, as illustrated in FIG. 4, by clustering the number of pixels N (= Hpix × Vpix) of the stained image into the designated number of classes N (< Hpix × Vpix), it is possible to suppress the redundancy of the processing time due to the enlargement of the matrix A.

In the clustering, spectra similar in a wavelength direction and an intensity direction among stained images are to be classified into the same class, for example. This will generate an image having a smaller number of pixels than the stained image, making it possible to reduce the scale of the matrix A' using this image as an input.

### (Image generation unit 133)

The image generation unit 133 is configured to generate image information based on the fluorescence spectrum separation result by the separation processing unit 132. For example, the image generation unit 133 can generate image information using fluorescence spectrum corresponding to one or a plurality of fluorescent substances, or can generate image information using autofluorescence spectrum corresponding to one or a plurality of autofluorescent substances. Note that the number and combination of fluorescent substances (molecules) or autofluorescent substances (molecules) used by the image generation unit 133 to generate image information are not particularly limited. Furthermore, when various types of processing (for example, segmentation, calculation of S/N value, or the like) using the fluorescence spectrum or the autofluorescence spectrum after separation have been performed, the image generation unit 133 may generate image information indicating a result of the processing.

### (Display unit 140)

The display unit 140 is configured to present the image information generated by the image generation unit 133 to the technician by displaying the image information on the display. Note that the type of display used as the display unit 140 is not particularly limited. Although not described in detail in the present embodiment, the image information generated by the image generation unit 133 may be presented to the technician by being projected by a projector or printed by a printer (in other words, a method of outputting the image information is not particularly limited).

### (Control unit 150)

The control unit 150 is a functional configuration that comprehensively controls overall processing performed by the information processing device 100. For example, the control unit 150 controls the start, end, and the like of various types of processing (for example, adjustment processing of the placement position of the fluorescence-stained specimen 30, emission processing of excitation light on the fluorescence-stained specimen 30, spectrum acquisition processing, generation processing of autofluorescence component correction image, color separation processing, generation processing of image information, display processing of image information, and the like) as described above based on an operation input by the technician performed via the operation unit 160. Note that the process to be controlled by the control unit 150 is not particularly limited. For example, the control unit 150 may control processing (for example, processing related to an operating system (OS)) generally performed in a general-purpose computer, a PC, a tablet PC, or the like.

### (1.5. Characteristic configuration of first embodiment)

The configuration example and the application example of the information processing system according to the present embodiment have been described as above. Next, a characteristic configuration of the present embodiment will be described in detail below with reference to the drawings.

As described above, in the present embodiment, in order to distinguish a fluorescence signal derived from an autofluorescent substance, which is considered to be a problem at imaging of a tissue sample, from a fluorescence signal derived from a fluorescent substance to be analyzed, a pseudo autofluorescence component image (autofluorescence component correction image) is generated using spectral information (autofluorescence component (spectrum)) derived from an autofluorescent substance obtained at the time of execution of color separation processing on the stained specimen image, so as to enable quantitative analysis of the stained specimen image using the generated pseudo autofluorescence component image.

More specifically, in the present embodiment, based on an autofluorescence component extracted from the stained specimen image, an autofluorescence component correction image is generated using the autofluorescence reference spectrum corresponding the autofluorescence component, and the stained specimen image is processed using the autofluorescence component correction image, thereby generating the fluorescence component image color-separated with higher accuracy. The generated fluorescence component image may be displayed on the display unit 140, or may undergo predetermined processing (analysis processing or the like) may be executed by the processing unit 130 or another configuration (for example, an analysis device or the like connected via a network). Note that the predetermined processing may be, for example, processing such as detection of a specific cell.

### (1.5.1. Basic operation example)

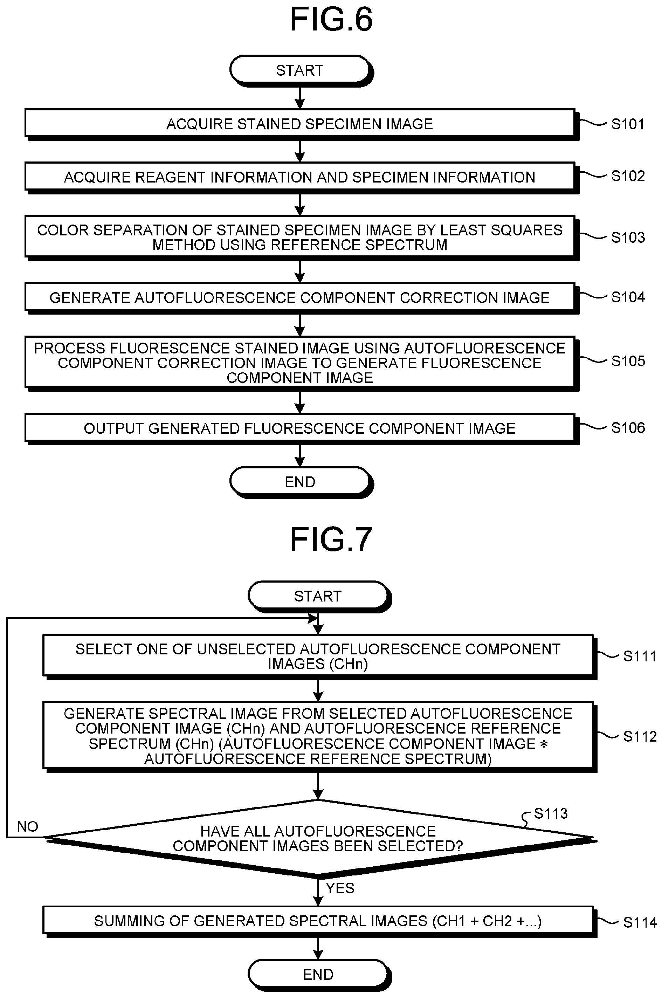

FIG. 6 is a flowchart illustrating a basic operation example of the information processing system according to the present embodiment. Note that the following operation is executed, for example, by each unit operating under the control of the control unit 150.

As illustrated in FIG. 6, in the first step of the basic operation according to the present embodiment, the information acquisition unit 111 of the acquisition unit 110 images the fluorescence-stained specimen 30 to acquire a stained specimen image (Step S101). The stained specimen image thus acquired is stored in the information storage unit 121 of the storage unit 120, for example.

In addition, the information acquisition unit 111 acquires reagent information and specimen information from the database 200 connected via the network (Step S102). As described above, here, the specimen information includes information regarding the autofluorescence reference spectrum of one or more autofluorescent substances in the specimen 20, and the reagent information includes information regarding the fluorescence reference spectrum of the fluorescent substance in the fluorescence-stained specimen 30. The acquired reagent information and specimen information are stored in the information storage unit 121 of the storage unit 120, for example.

Next, the separation processing unit 132 acquires the stained specimen image, the reagent information, and the specimen information stored in the information storage unit 121, and performs fitting of an autofluorescence reference spectrum using the least squares method, for example, on the acquired stained specimen image so as to execute color separation processing on the stained specimen image (Step S103). With the color separation processing, a fluorescence component image and an autofluorescence component image are generated.

Next, the separation processing unit 132 generates an autofluorescence component correction image using the autofluorescence component image generated in Step S103 and the autofluorescence reference spectrum included in the specimen information acquired in Step S102 (Step S104). The generation of the autofluorescence component correction image will be described below in more detail.

Next, the separation processing unit 132 generates a fluorescence component image by processing the stained specimen image using the autofluorescence component correction image (Step S105). In this manner, by removing the autofluorescence component included in the stained specimen image using the autofluorescence component correction image generated using the autofluorescence reference spectrum, it is possible to generate a fluorescence component image having a further improved color separation accuracy, that is, a further reduced residual amount of the autofluorescence component (correction unit).

The separation processing unit 132 then transmits the generated fluorescence component image to the image generation unit 133, an external server, or the like (Step S106). Thereafter, the present operation ends.

### (1.5.2. Example of autofluorescence component correction image generation flow)

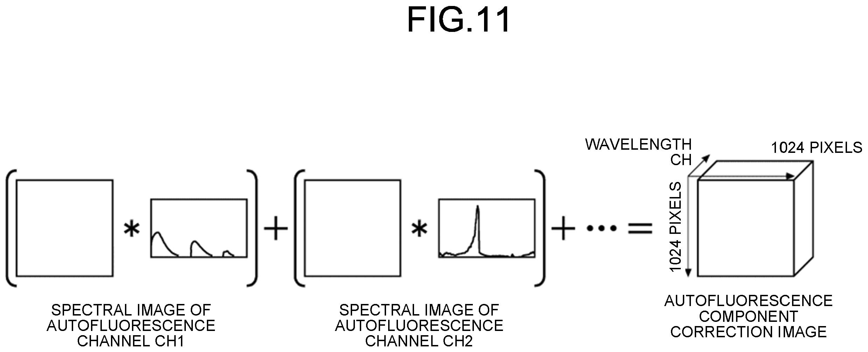

Next, an operation example when generating the autofluorescence component image described in Step S104 of FIG. 6 will be described. FIG. 7 is a flowchart illustrating an operation example when generating a pseudo autofluorescence component image according to the present embodiment. FIG. 8 is a view illustrating an example of the autofluorescence component image generated in Step S103 in FIG. 6, and FIG. 9 is a diagram illustrating an example of the autofluorescence reference spectrum included in the specimen information acquired in Step S102 in FIG. 6. FIG. 10 is a diagram illustrating Step S112 in FIG. 7, and FIG. 11 is a diagram illustrating Step S114 in FIG. 7.

As illustrated in FIG. 7, in the autofluorescence component correction image generation flow, the separation processing unit 132 first selects an unselected image (this is to be set as an autofluorescence component image of autofluorescence channel CHn (n is a natural number)) from among the autofluorescence component images (refer to FIG. 8) generated in Step S103 of FIG. 6 (Step S111). The autofluorescence channel may be identification information given for each autofluorescence.

Next, as illustrated in FIG. 10, the separation processing unit 132 generates a spectral image regarding the autofluorescence channel CHn based on the autofluorescence component image of the autofluorescence channel CHn selected in Step S111 and based on the autofluorescence reference spectrum of the autofluorescence channel CHn among the autofluorescence reference spectra (refer to FIG. 9) included in the specimen information acquired in Step S102 of FIG. 6 (Step S112). The above-described NMF can be used to generate the spectral image.

Next, the separation processing unit 132 determines whether all the autofluorescence component images have been selected in Step S111 (Step S113). In a case where all the autofluorescence component images have not been selected (NO in Step S113), the separation processing unit 132 returns to Step S111, selects an unselected autofluorescence component image, and executes subsequent operations.

In contrast, when all the autofluorescence component images have been selected in Step S111 (YES in Step S113), the separation processing unit 132 sums the spectral images of the individual autofluorescence channels CH generated in the iterated Step S112 as illustrated in FIG. 11 (Step S114). This result in generation of an autofluorescence component correction image. Thereafter, the separation processing unit 132 ends the operation illustrated in FIG. 7.

### (1.6. Extraction and analysis of analysis target region)

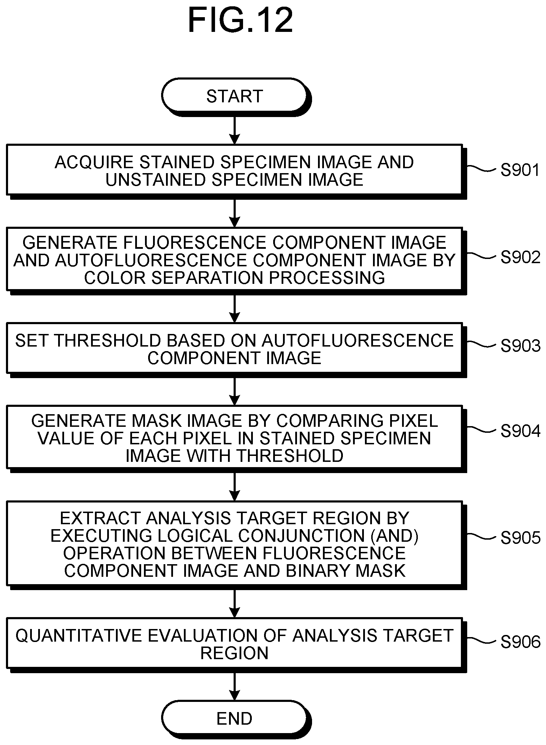

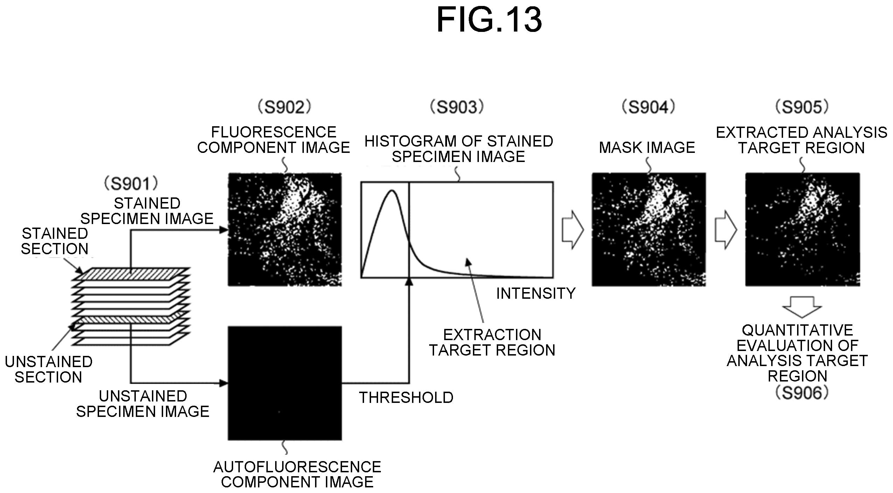

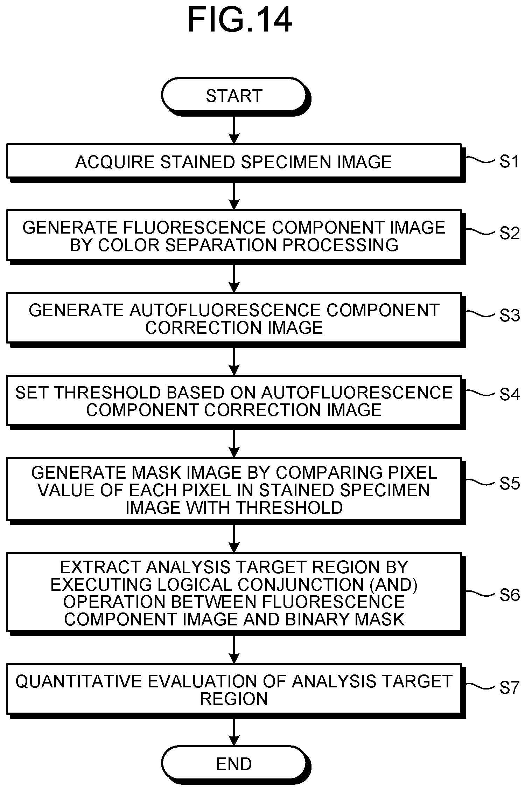

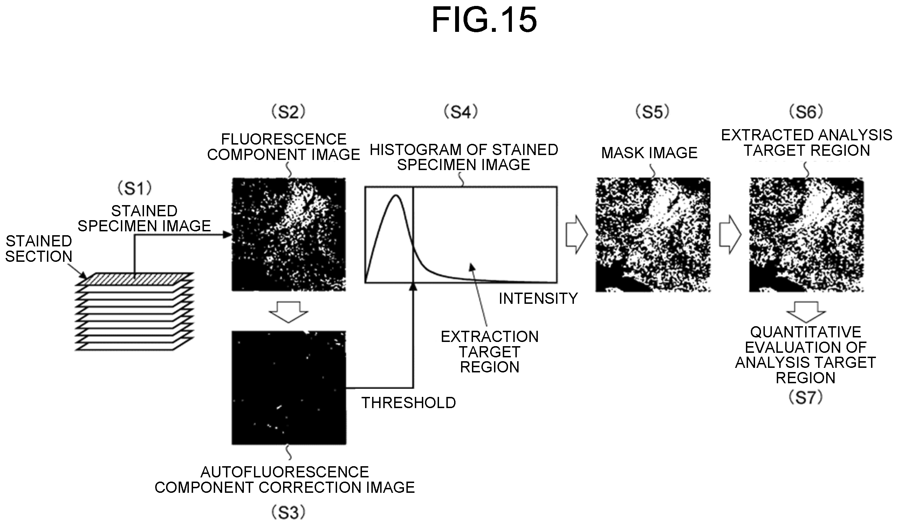

Next, extraction processing of an analysis target region from a fluorescence component image and analysis of the extracted region according to the present embodiment will be described using a comparative example. FIG. 12 is a flowchart illustrating extraction processing and analysis of an analysis target region according to the comparative example, and FIG. 13 is a schematic diagram illustrating extraction processing and analysis of an analysis target region according to the comparative example illustrated in FIG. 12. FIG. 14 is a flowchart illustrating extraction processing and analysis of an analysis target region according to the present embodiment, and FIG. 15 is a schematic diagram illustrating extraction processing and analysis of an analysis target region according to the present embodiment illustrated in FIG. 14.

### (Comparative example)

As illustrated in FIGS. 12 and 13, the comparative example executes a first step of acquiring a stained specimen image by imaging a stained section, while acquiring a captured image (hereinafter, referred to as an unstained specimen image) of an unstained section by imaging an unstained tissue section (hereinafter, also referred to as an unstained tissue section or an unstained section) adjacent to or close to the stained section (Step S901). Subsequently, color separation processing using the acquired stained specimen image and unstained specimen image is executed to generate a fluorescence component image and an autofluorescence component image (Step S902).

The next step is setting a threshold for the number of antibodies for extracting a region (extraction target region) that is a candidate for a region to be analyzed (analysis target region) based on the autofluorescence component image (Step S903). The threshold of the number of antibodies may be, for example, a threshold for a pixel value of each pixel in the fluorescence component image. The extraction target region may be a region hatched with dots in Step S903 of FIG. 13.

Next, the pixel value of each pixel in the stained specimen image is compared with the threshold, and a mask image for extracting the analysis target region is generated based on the comparison result (Step S904). This operation generates a binary mask, for example, in which '1' assigned set for the pixel value of a threshold or more and '0' is assigned for the pixel value less than the threshold, as the mask image. Note that the pixel or region to which '1' is assigned may be a candidate pixel or region to be analyzed, and the pixel or region to which '0' is assigned may be a pixel or region excluded from the analysis target.

Next, by executing a logical conjunction (AND) operation of each pixel of the mask image generated in Step S904 and each pixel of the fluorescence component image, an analysis target region is extracted from the fluorescence component image (Step S905). This operation makes it possible to obtain an extracted image in which a region (morphological information of an analysis target) labeled with the fluorescent dye antibody is extracted with a value extracted from the autofluorescence component image set as a threshold.

Thereafter, for example, the analysis target region is transferred to an analysis device such as an external server and quantitatively evaluated (Step S906).

### (Present embodiment)

On the other hand, as illustrated in FIGS. 14 and 15, the present embodiment executes a first step of acquiring a stained specimen image by imaging a stained section (Step S1). That is, in the present embodiment, there is no need to acquire an unstained specimen image by imaging an unstained section.

Next, in the present embodiment, a fluorescence component image is generated by executing color separation processing on the stained specimen image (Step S2). In the present embodiment, an autofluorescence component correction image is generated using the autofluorescence component image generated by the color separation processing and the autofluorescence reference spectrum included in the acquired specimen information (Step S3).

Next, a threshold of the number of antibodies for extracting an extraction target region, which is a candidate for an analysis target region, is set based on the autofluorescence component correction image (Step S4). Similarly to the description using FIGS. 12 and 13, the threshold of the number of antibodies may be, for example, a threshold for the pixel value of each pixel in the fluorescence component image, and the extraction target region may be a region hatched with dots in Step S4 of FIG. 15.

Thereafter, similarly to steps S904 to S906 in FIGS. 12 and 13, the pixel value of each pixel in the stained specimen image is compared with the threshold to generate a mask image (Step S5), and subsequently, the logical conjunction (AND) operation of the mask image generated in Step S5 and the fluorescence component image is executed to extract the analysis target region from the fluorescence component image (Step S6). This operation makes it possible to obtain an extracted image in which a region (morphological information of an analysis target) labeled with the fluorescent dye antibody is extracted with a value extracted from the autofluorescence component correction image set as a threshold. Subsequently, the extracted analysis target region is transferred to an analysis device such as an external server and quantitatively evaluated (Step S7).

### (1.7. Action and effects)

As described above, according to the present embodiment, in the analysis target region extraction processing, the user sets a value defined from the autofluorescence component correction image or a numerical value having objectivity as the threshold, and the analysis target region is extracted using a mask image (binary mask) generated based on the magnitude relationship between the pixel value in the stained specimen image and the threshold.

In this manner, by using the autofluorescence component correction image for setting the threshold, it is possible to omit labor of separately imaging an unstained section and acquiring an unstained specimen image, labor of generating an autofluorescence component image, and the like, and in addition, it is possible to set a threshold based on the autofluorescence component correction image that holds spatial information (signal distribution such as autofluorescence component and noise derived is the same) in the stained specimen image, in other words, that has a correlation with the stained specimen image or the fluorescence component image. This leads to an effect that it is possible to set the threshold having a correlation with the stained specimen image or the fluorescence component image.

### <2. Second embodiment>

Next, a second embodiment will be described. In the first embodiment described above, the thresholds for all the pixels of the stained specimen image are uniquely set from the generated autofluorescence component correction image. In the second embodiment, a threshold distribution image in which different, meaning not necessarily the same, thresholds are spatially distributed is generated by setting different thresholds for each pixel. Note that, in the present embodiment, since the configuration and the basic operation of the information processing system may be similar to the configuration and the basic operation of the information processing system according to the first embodiment, a detailed description thereof will be omitted here.

### (2.1. Extraction and analysis of analysis target region)

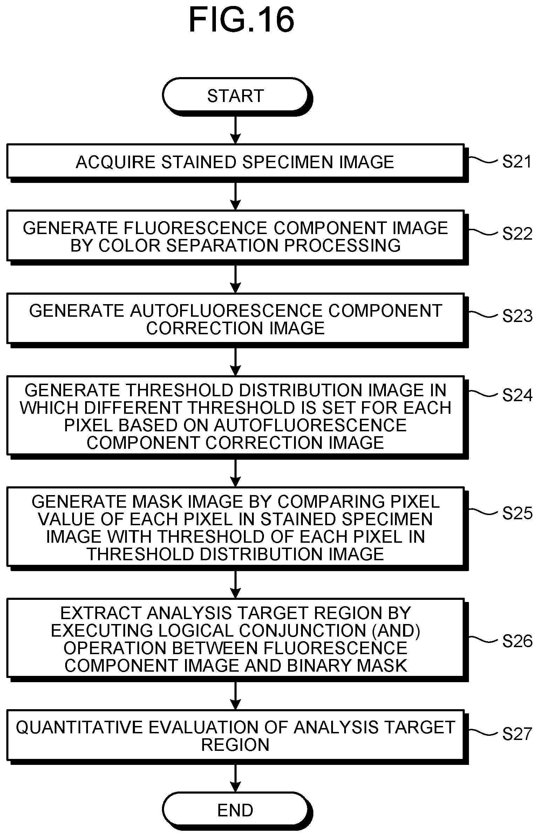

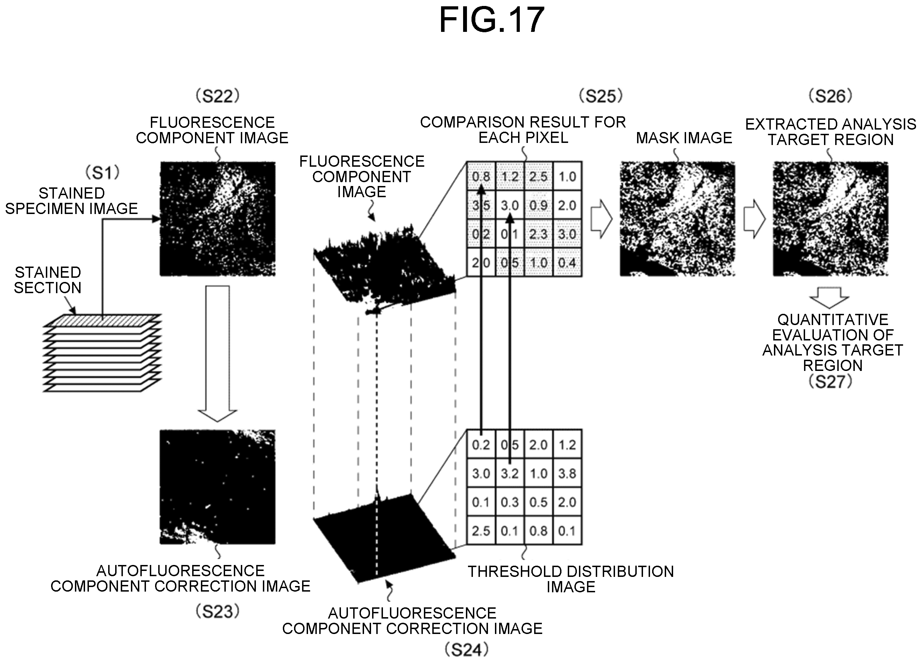

FIG. 16 is a flowchart illustrating extraction processing and analysis of an analysis target region according to the present embodiment, and FIG. 17 is a schematic diagram illustrating extraction processing and analysis of an analysis target region according to the present embodiment illustrated in FIG. 16.

As illustrated in FIGS. 16 and 17, the present embodiment first performs operations similarly to the operations described using steps S1 to S3 in FIGS. 14 and 15 in the first embodiment, such that a stained specimen image is acquired by capturing a stained section (Step S21), a fluorescence component image is generated by executing color separation processing on the stained specimen image (Step S22), and an autofluorescence component correction image is generated using the autofluorescence component image generated by the color separation processing and using the autofluorescence reference spectrum included in the acquired specimen information (Step S23).

Next, in the present embodiment, a threshold distribution image in which a threshold for the number of antibodies, which differs for each pixel, is set is generated based on the autofluorescence component correction image (Step S24). As a method of generating the threshold distribution image having different thresholds for each pixel, for example, it is possible to adopt a method of multiplying the pixel value of each pixel of the autofluorescence component correction image by a preset coefficient. Here, the coefficient may be determined within a numerical range considered to be appropriate, for example. As a specific example, it is allowable to use a value based on the maximum value or the average value of the pixel values in the autofluorescence component correction image, as the coefficient.

Next, in the present embodiment, the pixel value of each pixel in the stained specimen image is compared with the threshold of the corresponding pixel in the threshold distribution image, and a mask image for extracting the analysis target region is generated based on the comparison result (Step S25). This operation generates a binary mask, for example, in which '1' assigned set for the pixel value of a threshold or more and '0' is assigned for the pixel value less than the threshold, as the mask image. Note that, similarly to the first embodiment, the pixel or region to which '1' is assigned may be a candidate pixel or region to be analyzed, and the pixel or region to which '0' is assigned may be a pixel or region excluded from the analysis target. Furthermore, in the comparison result for each pixel illustrated in Step S25 of FIG. 17, the pixel hatched with a dot may be a pixel having a pixel value being a corresponding threshold or more, for example.

Thereafter, similarly to steps S6 to S7 in FIGS. 14 and 15, the logical conjunction (AND) operation of the mask image generated in S25 and the fluorescence component image is executed to extract the analysis target region from the fluorescence component image (Step S26). This operation makes it possible to obtain an extracted image in which a region (morphological information of an analysis target) labeled with the fluorescent dye antibody is extracted with a value extracted from the autofluorescence component correction image set as a threshold. Subsequently, the extracted analysis target region is transferred to an analysis device such as an external server and quantitatively evaluated (Step S27).

### (2.2. Action and effects)

As described above, in generating the binary mask for extracting only the target region from the stained specimen image in the present embodiment, the threshold distribution image allowing different thresholds to be spatially distributed is generated, instead of uniquely setting the threshold from the autofluorescence component correction image as in the first embodiment. This makes it possible to set the threshold using the pixel value of the autofluorescence component correction image corresponding to the stained specimen image, leading to achievement of extraction of the analysis target region using the threshold distribution image that holds spatial information of noise that can occur in the system, such as the distribution of the autofluorescence component for each tissue region and the influence of hardware. This makes it possible to further improve the analysis accuracy in fluorescence microscopy.

Since other configurations, operations, and effects may be similar to those in the above-described embodiment, detailed description thereof will be omitted here.

### <3. Third embodiment>

Next, a third embodiment will be described. The above embodiment has described an exemplary case of generating the autofluorescence component correction image from the stained specimen image. In contrast, present embodiment generates a pseudo fluorescence component image (hereinafter, referred to as a fluorescence component correction image) in addition to the autofluorescence component correction image, and calculates a spectral intensity ratio of these images to acquire the ratio of the fluorescent dye intensity to the autofluorescence as information. Note that, in the present embodiment, since the configuration and the basic operation of the information processing system may be similar to the configuration and the basic operation of the information processing system according to the above embodiment, a detailed description thereof will be omitted here.

### (3.1. Generation and analysis of spectral intensity ratio image)

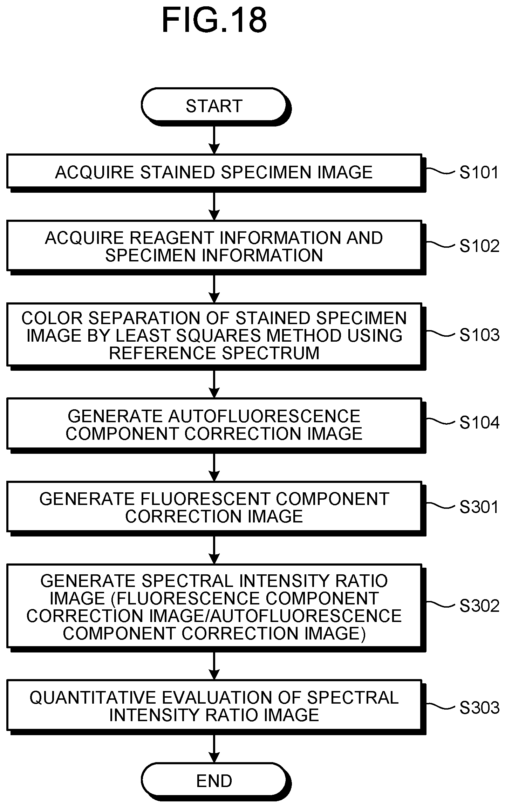

In the present embodiment, the spectral intensity ratio is generated as spatially distributed information, that is, image data (spectral intensity ratio image), for example. FIG. 18 is a flowchart illustrating generation processing and analysis of a spectral intensity ratio image according to the present embodiment.

As illustrated in FIG. 18, the present embodiment first executes operations similar to steps S101 to S104 of FIG. 6 in the first embodiment to generate an autofluorescence component correction image (Step S104).

Next, in the present embodiment, for example, the fluorescence component correction image is generated by applying the operation similar to that used in generating the autofluorescence component correction image (Step S301). Note that the fluorescence component correction image generation flow can be performed by replacing the autofluorescence component image in the operation described with reference to FIG. 7 in the first embodiment with the fluorescence component image and replacing the autofluorescence reference spectrum with the fluorescence reference spectrum, and thus, a detailed description thereof is omitted here.

Next, the separation processing unit 132 calculates a ratio (spectral intensity ratio) between corresponding pixels in the fluorescence component correction image and the autofluorescence component correction image to generate a spectral intensity ratio image (Step S302).

Thereafter, for example, the spectral intensity ratio image is transferred to an analysis device such as an external server and quantitatively evaluated (Step S303).

### (3.2. Method of generating spectral intensity image)

In the present embodiment, the spectral intensity ratio image may be generated, for example, by individually generating a spectral intensity image indicating the spectral intensity of each pixel of the fluorescence component correction image and a spectral intensity image indicating the spectral intensity of each pixel of the autofluorescence component correction image, and then calculating the ratio of the spectral intensities of the corresponding pixels.

Here, a method of generating the spectral intensity image of each of the fluorescence component correction image and the autofluorescence component correction image is not particularly limited. Two exemplary generation methods will be described below.

### (3.2.1 First generation method)

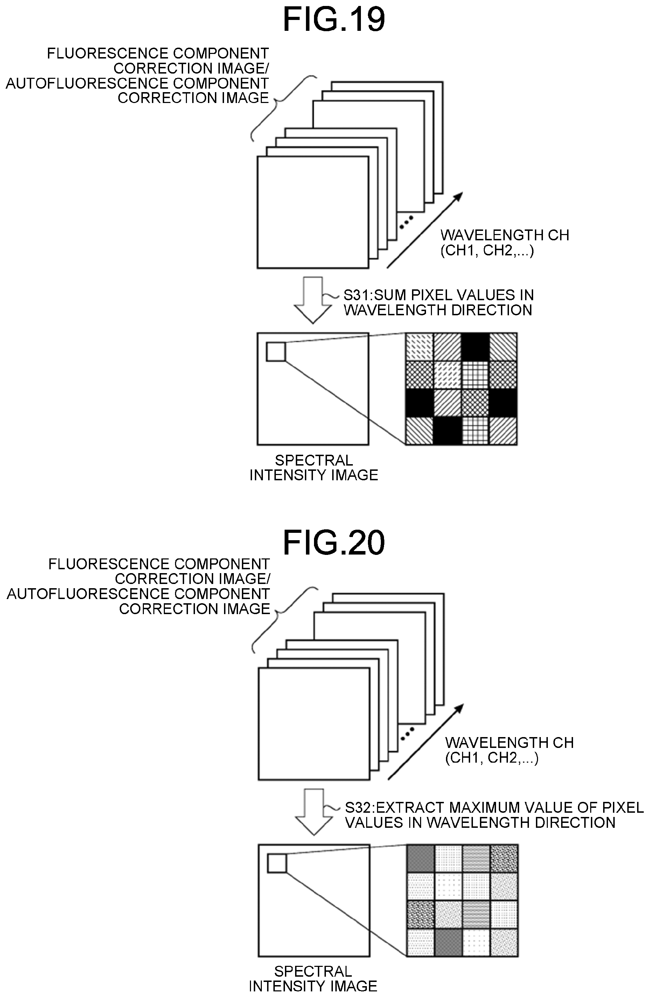

FIG. 19 is a diagram illustrating a first generation method. As illustrated in FIG. 19, the first generation method is a method of generating a spectral intensity image by summing, in the wavelength direction, the pixel values for each wavelength channel of the fluorescence component correction image/autofluorescence component correction image having a data cube structure (Step S31).

### (3.2.2 Second generation method)

FIG. 20 is a diagram illustrating a second generation method. As illustrated in FIG. 20, the second generation method is a method of generating the spectral intensity image by extracting a pixel value having the maximum value in the wavelength direction from among the pixel values for each wavelength channel of the fluorescence component correction image/autofluorescence component correction image having a data cube structure (Step S32).

### (3.3. Action and effects)

As described above, in the present embodiment, the fluorescence component correction image is generated also by multiplying the fluorescence component by the fluorescence reference spectrum extracted by the color separation processing for each pixel of the fluorescence component image, so as to obtain the spectral intensity image of each of the fluorescence component correction image and the autofluorescence component correction image. In addition, a spectral intensity ratio image, which is information spatially indicating the ratio of the fluorescent dye intensity to the autofluorescence, is generated from the spectral intensity ratio between each pixel of the fluorescence component correction image and each pixel of the autofluorescence component correction image. The spectral intensity ratio image thus generated can be utilized for the purposes such as performance evaluation of a measurement system (corresponding to the acquisition unit 110) in the information processing system, color separation accuracy evaluation in the processing unit 130, and design/evaluation of a fluorescent reagent panel.

Furthermore, according to the present embodiment, it is possible, for example, to store the spectral intensity ratio image generated as described above in the storage unit 120 or the like in association with image acquisition conditions such as an image capture condition when the stained specimen image is acquired by the acquisition unit 110, and a labeling condition (staining condition) of the fluorescent reagent 10 for the specimen 20, so as to be utilized as reference information when acquiring a stained specimen image using the same acquisition unit 110. In addition, the spectral intensity ratio image and the image acquisition condition can be stored in a server on a network and shared the spectral intensity ratio image and the image acquisition condition with another information processing system or the information processing device 100 so as to be utilized as reference information when acquiring a stained specimen image using the acquisition unit 110 of the same model in another information processing system. Specifically, for example, the spectral intensity ratio images and the image acquisition conditions accumulated in the storage unit 120, the server, and the like may be utilized for inter-device baseline correction, calibration curve correction, and the like in the case of using the acquisition unit 110 of the same model.

Since other configurations, operations, and effects may be similar to those in the above-described embodiment, detailed description thereof will be omitted here.

### <4. Fourth embodiment>

Next, a fourth embodiment will be described. In the above-described embodiment, for example, NMF, SVD, PCA, or the like is used for extracting the autofluorescence spectrum and/or the fluorescence spectrum. In contrast, the present embodiment will describe a case where an autofluorescence spectrum and/or a fluorescence spectrum is extracted using machine learning instead of these techniques. Note that, in the present embodiment, since the configuration and the basic operation of the information processing system may be similar to the configuration and the basic operation of the information processing system according to the above embodiment, a detailed description thereof will be omitted here.

### (4.1. Generation of fluorescence component image using machine learning)

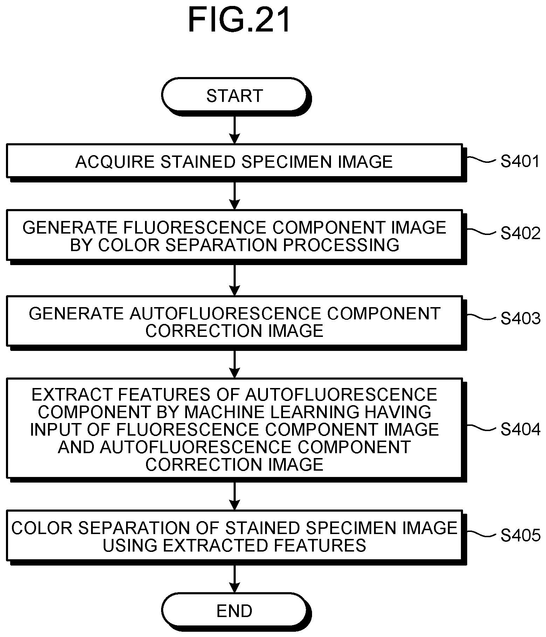

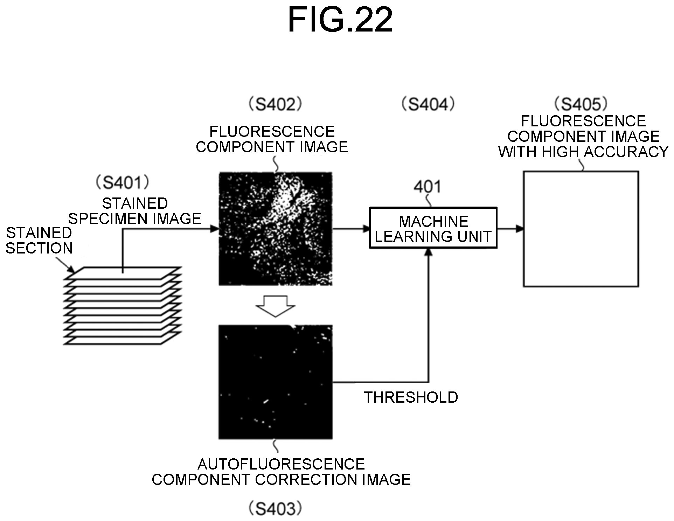

FIG. 21 is a flowchart for describing processing of generating a fluorescence component image using machine learning according to the present embodiment, and FIG. 22 is a schematic diagram illustrating processing of generating a fluorescence component image using machine learning according to the present embodiment illustrated in FIG. 21.

As illustrated in FIGS. 21 and 22, the present embodiment first executes operations similar to steps S1 to S3 of FIG. 14 in the second embodiment to generate an autofluorescence component correction image (steps S401 to S403).

Next, in the present embodiment, the fluorescence component image generated by the color separation processing in Step S401 and the pseudo autofluorescence component image generated in Step S403 are input to a machine learning unit 401. Subsequently, the machine learning unit 401 executes deep learning as unsupervised learning using the fluorescence component image and the pseudo autofluorescence component image as input images, thereby extracting features (for example, autofluorescence spectra) such as signal strength and distribution derived from the autofluorescence component from the stained specimen image (Step S404). It is possible to apply, as the deep learning, various types of machine learning such as deep neural network (DNN), convolutional neural network (CNN), or recurrent neural network (RNN). Furthermore, the machine learning unit 401 may be installed on the processing unit 130, etc. in the information processing device 100, for example, or may be installed on a cloud server or the like connected to the information processing device 100 via a predetermined network.

In the present embodiment, a next step (Step S405) is to generate a fluorescence component image with higher accuracy with further reduced residual amount of the autofluorescence component using the features extracted in Step S404, ending the present operation.

### (4.2. Action and effects)

As described above, the present embodiment applies machine learning in the form of unsupervised learning using the fluorescence component image generated by the color separation processing for the stained specimen image and the autofluorescence component correction image generated from the fluorescence component image, as input images, and extracts features such as signal strength and distribution derived from the autofluorescence component from the stained specimen image, and then generates a fluorescence component image with higher accuracy based on the extracted features. In this manner, by executing machine learning based on the stained specimen image, in other words, using the autofluorescence component correction image generated from the stained specimen image as an input, it is possible to facilitate association between the input image and the output image of the machine learning, leading to an effect of facilitation of enhancing the learning effect.

Since other configurations, operations, and effects may be similar to those in the above-described embodiment, detailed description thereof will be omitted here.

### <5. Configuration example of measurement system>

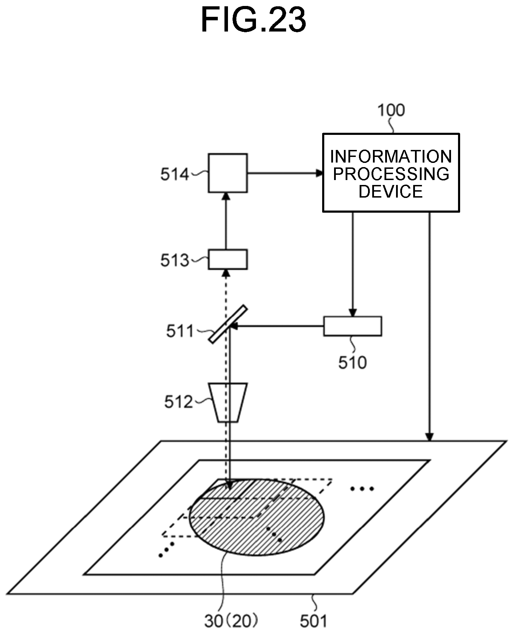

Next, a configuration example of a measurement system in the information processing device 100 according to the above-described embodiment(s) (hereinafter, simply referred to as embodiment(s)) will be described. FIG. 23 is a diagram illustrating an example of a measurement system of the information processing system according to the embodiment. Note that FIG. 23 illustrates an example of a measurement system for performing wide-field imaging of the fluorescence-stained specimen 30 (or the specimen 20 that is an unstained specimen) using a technique such as Whole Slide Imaging (WSI). However, the measurement system according to the embodiment is not limited to the measurement system illustrated in FIG. 23, and may be variously modified as long as it is a measurement system capable of acquiring image data with sufficient resolution of the entire imaging region or the region of interest (hereinafter, referred to as wide-field image data) such as a measurement system that captures the entire imaging region or a necessary region (also referred to as a region of interest) of the entire imaging region or the region of interest at a time, or a measurement system that acquires an image of the entire imaging region or the region of interest by line scanning.

As illustrated in FIG. 23, the measurement system according to the embodiment includes, for example, an information processing device 100, an XY stage 501, an excitation light source 510, a beam splitter 511, an objective lens 512, a spectroscope 513, and a photodetector 514.

The XY stage 501 is a stage on which the fluorescence-stained specimen 30 (or specimen 20) to be analyzed is placed, and may be a stage movable in a plane (XY plane) parallel to the placement surface of the fluorescence-stained specimen 30 (or specimen 20), for example.

The excitation light source 510 is a light source for exciting the fluorescence-stained specimen 30 (or specimen 20), and emits a plurality of excitation light beams having different wavelengths along a predetermined optical axis, for example.

The beam splitter 511 includes parts such as a dichroic mirror, for example, reflects excitation light from the excitation light source 510, and transmits fluorescence from the fluorescence-stained specimen 30 (or specimen 20).

The objective lens 512 applies the excitation light reflected by the beam splitter 511 to the fluorescence-stained specimen 30 (or specimen 20) on the XY stage 501.

The spectroscope 513 is formed with one or more prisms, lenses, and the like, and splits the fluorescence emitted from the fluorescence-stained specimen 30 (or specimen 20) and transmitted through the objective lens 512 and the beam splitter 511 in predetermined directions.

The photodetector 514 detects light intensity for each wavelength of fluorescence split by the spectroscope 513, and inputs a fluorescence signal (fluorescence spectrum and/or autofluorescence spectrum) obtained by the detection to the fluorescence signal acquisition unit 112 of the information processing device 100.

In the configuration as described above, in a case where the entire imaging region exceeds a region that can be imaged in one time (hereinafter, referred to as a visual field) as in the WSI, each visual field is sequentially imaged by moving the XY stage 501 for each imaging. Subsequently, by tiling the image data obtained by imaging each visual field (hereinafter, referred to as visual field image data), the wide-field image data of the entire imaging region is generated. The generated wide-field image data is stored in the fluorescence signal storage unit 122, for example. Tiling of the visual field image data may be executed in the acquisition unit 110 of the information processing device 100, may be executed in the storage unit 120, or may be executed in the processing unit 130.

By executing the above-described processing on the obtained wide-field image data, the processing unit 130 according to the embodiment acquires a coefficient C, that is, a fluorescence separation image for each fluorescent molecule (or an autofluorescence separation image for each autofluorescent molecule).

### <6. Method of calculating number of fluorescent molecules (or number of antibodies)>

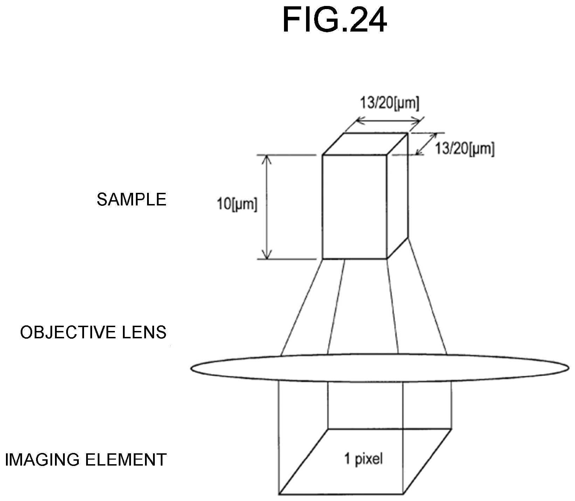

Next, a method of calculating the number of fluorescent molecules (or the number of antibodies) in one pixel in the above-described embodiment will be described. FIG. 24 is a schematic diagram illustrating a method of calculating the number of fluorescent molecules (or the number of antibodies) in one pixel in the embodiment. In the example illustrated in FIG. 24, in a case where the imaging element and the sample are arranged across the objective lens, the size of the bottom surface of the sample corresponding to the imaging element 1 [pixel] is assumed to be 13/20 (µm) × 13/20 (µm). The thickness of the sample is assumed to be 10 (µm). In this case, the volume (m³) of the cuboid is expressed by 13/20 (µm) × 13/20 (µm) × 10 (µm). The volume (liter) is represented by 13/20 (µm) × 13/20 (µm) × 10 (µm) × 10³.

Assuming that the concentration (density) of the number of antibodies (which may be the number of fluorescent molecules) contained in the sample is uniform and is 300 (nM), the number of antibodies per pixel is represented by the following Formula (11). $300 * {10}^{- 9} * \left(\frac{13}{20}*\frac{13}{20}*10*{\left({10}^{- 6}\right)}^{3}\right) * {10}^{3} * 6.02 * {10}^{23}$ (11)

In this manner, the number of fluorescent molecules or the number of antibodies in the fluorescence-stained specimen 30 is calculated as a result of the fluorescence separation processing, whereby the technician can compare the number of fluorescent molecules among a plurality of fluorescent substances or compare pieces of data imaged under different conditions with each other. Furthermore, since the number of fluorescent molecules or the number of antibodies is a discrete value while the luminance (or fluorescence intensity) is a continuous value, the information processing device 100 according to the modification can reduce the data volume by outputting image information based on the number of fluorescent molecules or the number of antibodies.

### <7. Hardware configuration example>

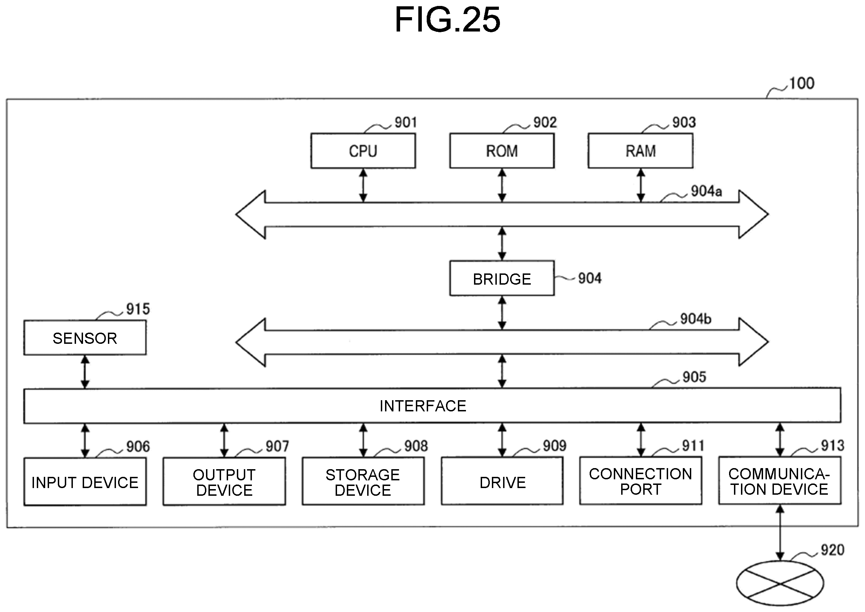

The embodiments of the present disclosure and their modifications have been described above. Next, a hardware configuration example of the information processing device 100 according to each embodiment and modifications will be described with reference to FIG. 25. FIG. 25 is a block diagram illustrating a hardware configuration example of the information processing device 100. Various processes by the information processing device 100 are implemented by cooperative operations of software and hardware described below.

As illustrated in FIG. 25, an information processing device 100 includes a central processing unit (CPU) 901, read only memory (ROM) 902, random access memory (RAM) 903, and a host bus 904a. The information processing device 100 further includes a bridge 904, an external bus 904b, an interface 905, an input device 906, an output device 907, a storage device 908, a drive 909, a connection port 911, a communication device 913, and a sensor 915. The information processing device 100 may include a processing circuit such as a DSP or an ASIC instead of or in addition to the CPU 901.

The CPU 901 functions as an arithmetic processing device and a control device, and controls the overall operation in the information processing device 100 according to various programs. In addition, the CPU 901 may be a microprocessor. The ROM 902 stores programs and calculation parameters used by the CPU 901. The RAM 903 temporarily stores a program used in the execution of the CPU 901, parameters that change appropriately in the execution, or the like. The CPU 901 can implement, for example, at least the processing unit 130 and the control unit 150 of the information processing device 100.

The CPU 901, ROM 902, and RAM 903 are connected to each other by the host bus 904a including a CPU bus or the like. The host bus 904a is connected to the external bus 904b such as a Peripheral Component Interconnect/Interface (PCI) bus via the bridge 904. There is no need to separate the host bus 904a, the bridge 904, and the external bus 904b from each other, and these functions may be implemented on one bus.

The input device 906 is actualized by a device to which the technician inputs information, such as a mouse, a keyboard, a touch panel, a button, a microphone, a switch, and a lever. Furthermore, the input device 906 may be, for example, a remote control device using infrared rays or other radio waves, or an externally connected device such as a mobile phone or a PDA that supports the operation of the information processing device 100. Furthermore, the input device 906 may include, for example, an input control circuit that generates an input signal based on the information input by the technician using the above input means and outputs the input signal to the CPU 901. By operating the input device 906, the technician can input various data to the information processing device 100 and give an instruction on the processing operation. The input device 906 can implement at least the operation unit 160 of the information processing device 100, for example.

The output device 907 is formed by a device capable of visually or audibly notifying the technician of acquired information. Examples of such devices include display devices such as CRT display devices, liquid crystal display devices, plasma display devices, EL display devices, and lamps, audio output devices such as speakers and headphones, and printer devices. The output device 907 can implement at least the display unit 140 of the information processing device 100, for example.

The storage device 908 is a device for storing data. The storage device 908 is implemented by, for example, a magnetic storage device such as an HDD, a semiconductor storage device, an optical storage device, an optical magnetic storage device, or the like. The storage device 908 may include a storage medium, a recording device that records data on the storage medium, a reading device that reads data from the storage medium, a deleting device that deletes the data recorded on the storage medium, and the like. This storage device 908 stores programs executed by the CPU 901, various data, as well as various data acquired from the outside, and the like. The storage device 908 can implement at least the storage unit 120 of the information processing device 100, for example.

The drive 909 is a reader/writer for a storage medium, and is built in or externally connected to the information processing device 100. The drive 909 reads information recorded on a removable storage medium such as a mounted magnetic disk, optical disk, magneto-optical disk, or semiconductor memory, and outputs the read information to the RAM 903. The drive 909 can also write information to the removable storage medium.

The connection port 911 is an interface connected to an external device, and is a connecting port to an external device capable of transmitting data by, for example, a universal serial bus (USB).

The communication device 913 is, for example, a communication interface formed by a communication device or the like for connecting to a network 920. The communication device 913 is, for example, a communication card for wired or wireless Local Area Network (LAN), Long Term Evolution (LTE), Bluetooth (registered trademark), Wireless USB (WUSB), or the like. Furthermore, the communication device 913 may be a router for optical communication, an Asymmetric Digital Subscriber Line (ADSL) router, a modem for various communications, or the like. The communication device 913 can transmit and receive signals or the like to and from the Internet and other communication devices in accordance with a predetermined protocol such as TCP/IP.

In the present embodiment, the sensor 915 includes a sensor capable of acquiring a spectrum (for example, an imaging element or the like), and may further include other sensors (for example, an acceleration sensor, a gyro sensor, a geomagnetic sensor, a pressure-sensitive sensor, a sound sensor, a ranging sensor, or the like). The sensor 915 can implement at least the fluorescence signal acquisition unit 112 of the information processing device 100, for example.

The network 920 is a wired or wireless transmission path for information transmitted from a device connected to the network 920. For example, the network 920 may include a public network such as the Internet, a telephone network, and a satellite communication network, or various local area networks (LANs) including Ethernet (registered trademark), wide area networks (WANs), or the like. Furthermore, the network 920 may include a dedicated network such as an Internet protocol-virtual private network (IP-VPN).

The hardware configuration example capable of implementing the functions of the information processing device 100 has been described above. Each of the above-described components may be implemented by using a general-purpose member, or may be implemented by hardware devices specialized for the function of individual components. Accordingly, it is possible to appropriately change the hardware configuration to be used according to the technical level at the time of conducting the present disclosure.

Incidentally, it is possible to create a computer program for implementation of individual functions of the information processing device 100 as above and possible to install the created program on a PC or the like. Furthermore, it is also possible to provide a computer-readable recording medium storing such a computer program. Examples of the recording medium include a magnetic disk, an optical disk, a magneto-optical disk, a flash drive, or the like. Furthermore, the computer program described above may be distributed via a network, for example, without using a recording medium.

The preferred embodiments of the present disclosure have been described in detail above with reference to the accompanying drawings. However, the technical scope of the present disclosure is not limited to such examples. It will be apparent to those skilled in the art of the present disclosure that various modifications and alterations can be conceived within the scope of the technical idea described in the claims and naturally fall within the technical scope of the present disclosure.

Furthermore, the effects described in the present specification are merely illustrative or exemplary and are not limited. That is, the technology according to the present disclosure can exhibit other effects that are apparent to those skilled in the art from the description of the present specification in addition to or instead of the above effects.

Note that the following configurations also belong to the technical scope of the present disclosure.
(1) An information processing device including:
   a separation unit that separates a fluorescence image obtained by observing a specimen labeled with one or more fluorescent dyes into a fluorescence component image containing one or more fluorescence components and an autofluorescence component image containing one or more autofluorescence components;
   a generation unit that generates an autofluorescence component correction image using a reference spectrum of each of one or more autofluorescent substances included in the specimen and using the autofluorescence component image; and
   a processing unit that processes the fluorescence component image based on the autofluorescence component correction image.
(2) The information processing device according to (1),
   wherein the processing unit generates a fluorescence component correction image obtained by correcting the fluorescence component image using the autofluorescence component correction image.
(3) The information processing device according to (1) or (2),
   wherein the generation unit generates the autofluorescence component correction image by summing a result of multiplying the autofluorescence component image and the reference spectrum for each of the autofluorescent substances.
(4) The information processing device according to any one of (1) to (3),
   wherein the processing unit generates region information indicating a region labeled with the fluorescent dye from the autofluorescence component correction image, and processes the fluorescence component image using the generated region information.
(5) The information processing device according to (4),
   wherein the region information is a binary mask indicating a region labeled with the fluorescent dye.
(6) The information processing device according to (5),
   wherein the processing unit generates the binary mask based on a magnitude relationship between a threshold set based on the autofluorescence component correction image and a pixel value of each pixel in the fluorescence image.
(7) The information processing device according to (5),
   wherein the processing unit sets a threshold for each of the pixels based on a pixel value of each of the pixels in the autofluorescence component correction image, and generates the binary mask based on a magnitude relationship between the threshold for each of the pixels and a pixel value of each of the pixels in the fluorescence image.
(8) The information processing device according to any one of (1) to (7), wherein the generation unit further generates a fluorescence component correction image by using a reference spectrum of each of the one or more fluorescent dyes and the fluorescence component image.
(9) The information processing device according to (8), further including an evaluation unit that evaluates at least one of: measurement accuracy of a measurement system that acquires the fluorescence image; performance of separation between the fluorescence component image and the autofluorescence component image performed by the separation unit; and staining performance of a fluorescent reagent panel including the one or more fluorescent dyes, based on the fluorescence component correction image and the autofluorescence component correction image.
(10) The information processing device according to (8) or (9), further including:
   an image generation unit that generates a spectral intensity ratio image representing a ratio between spectral intensity of each pixel in the fluorescence component correction image and spectral intensity of each pixel in the autofluorescence component correction image; and
   a storage unit that stores an imaging condition at a time of acquisition of the fluorescence image by imaging the specimen, and the spectral intensity ratio image, in association with each other.
(11) The information processing device according to any one of (1) to (10), further including a correction unit that corrects the fluorescence component image based on the fluorescence image, the fluorescence component image, and the autofluorescence component correction image,
   wherein the processing unit processes the corrected fluorescence component image based on the autofluorescence component correction image.
(12) The information processing device according to (11),
   wherein the correction unit estimates features of the autofluorescent substance in the fluorescence image from the fluorescence component image and the autofluorescence component correction image, and corrects the fluorescence component image based on the estimated features.
(13) The information processing device according to (12),
   wherein the correction unit estimates the features by using a trained model having the fluorescence component image and the autofluorescence component correction image as inputs.
(14) The information processing device according to (13),
   wherein the trained model is a model trained by using unsupervised learning.
(15) The information processing device according to any one of (1) to (14), further including an acquisition unit that acquires the fluorescence image by imaging the specimen.
(16) The information processing device according to any one of (1) to (15),
   wherein the processing unit detects a specific cell included in the fluorescence image based on the processed fluorescence component image.
(17) An information processing method including:
   separating a fluorescence image obtained by observing a specimen labeled with one or more fluorescent dyes into a fluorescence component image containing one or more fluorescence components and an autofluorescence component image containing one or more autofluorescence components;
   generating an autofluorescence component correction image using a reference spectrum of each of one or more autofluorescent substances included in the specimen and using the autofluorescence component image; and
   processing the fluorescence component image based on the autofluorescence component correction image.
(18) A program for causing a computer to execute processes including:
   a process of separating a fluorescence image obtained by observing a specimen labeled with one or more fluorescent dyes into a fluorescence component image containing one or more fluorescence components and an autofluorescence component image containing one or more autofluorescence components;
   a process of generating an autofluorescence component correction image using a reference spectrum of each of one or more autofluorescent substances included in the specimen and using the autofluorescence component image; and
   a process of processing the fluorescence component image based on the autofluorescence component correction image.
(19) A microscope system including:
   a light source that emits light to a specimen labeled with one or more fluorescent dyes; an imaging device that observes fluorescence emitted from the specimen irradiated with the light; and a program designed to execute processes on a fluorescence image acquired by the imaging device,
   wherein the program, by being run on a computer, causes the computer to execute processes including:
      a process of separating the fluorescence image into a fluorescence component image including one or more fluorescence components and an autofluorescence component image including one or more autofluorescence components;
      a process of generating an autofluorescence component correction image using a reference spectrum of each of one or more autofluorescent substances included in the specimen and the autofluorescence component image; and
      a process of processing the fluorescence component image based on the autofluorescence component correction image.
(20) An analysis system including:
   the information processing device according to (1); and
   an analysis device that is connected to the information processing device via a predetermined network and analyzes the fluorescence component image processed by the information processing device.

### Reference Signs List

- 10: FLUORESCENT REAGENT
- 11: REAGENT IDENTIFICATION INFORMATION
- 20: SPECIMEN
- 21: SPECIMEN IDENTIFICATION INFORMATION
- 30: FLUORESCENCE-STAINED SPECIMEN
- 100: INFORMATION PROCESSING DEVICE
- 110: ACQUISITION UNIT
- 111: INFORMATION ACQUISITION UNIT
- 112: FLUORESCENCE SIGNAL ACQUISITION UNIT
- 120: STORAGE UNIT
- 121: INFORMATION STORAGE UNIT
- 122: FLUORESCENCE SIGNAL STORAGE UNIT
- 130: PROCESSING UNIT
- 132: SEPARATION PROCESSING UNIT
- 133: IMAGE GENERATION UNIT
- 140: DISPLAY UNIT
- 150: CONTROL UNIT
- 160: OPERATION UNIT
- 200: DATABASE
- 401: MACHINE LEARNING UNIT

### CLAUSES

1. An information processing device including:
   a separation unit that separates a fluorescence image obtained by observing a specimen labeled with one or more fluorescent dyes into a fluorescence component image containing one or more fluorescence components and an autofluorescence component image containing one or more autofluorescence components;
   a generation unit that generates an autofluorescence component correction image using a reference spectrum of each of one or more autofluorescent substances included in the specimen and using the autofluorescence component image; and
   a processing unit that processes the fluorescence component image based on the autofluorescence component correction image.
2. The information processing device according to clause 1,
   wherein the processing unit generates a fluorescence component correction image obtained by correcting the fluorescence component image using the autofluorescence component correction image.
3. The information processing device according to clause 1,
   wherein the generation unit generates the autofluorescence component correction image by summing a result of multiplying the autofluorescence component image and the reference spectrum for each of the autofluorescent substances.
4. The information processing device according to clause 1,
   wherein the processing unit generates region information indicating a region labeled with the fluorescent dye from the autofluorescence component correction image, and processes the fluorescence component image using the generated region information.
5. The information processing device according to clause 4,
   wherein the region information is a binary mask indicating a region labeled with the fluorescent dye.
6. The information processing device according to clause 5,
   wherein the processing unit generates the binary mask based on a magnitude relationship between a threshold set based on the autofluorescence component correction image and a pixel value of each pixel in the fluorescence image.
7. The information processing device according to clause 5,
   wherein the processing unit sets a threshold for each of the pixels based on a pixel value of each of the pixels in the autofluorescence component correction image, and generates the binary mask based on a magnitude relationship between the threshold for each of the pixels and a pixel value of each of the pixels in the fluorescence image.
8. The information processing device according to clause 1, wherein the generation unit further generates a fluorescence component correction image by using a reference spectrum of each of the one or more fluorescent dyes and the fluorescence component image.
9. The information processing device according to clause 8, further including an evaluation unit that evaluates at least one of: measurement accuracy of a measurement system that acquires the fluorescence image; performance of separation between the fluorescence component image and the autofluorescence component image performed by the separation unit; and staining performance of a fluorescent reagent panel including the one or more fluorescent dyes, based on the fluorescence component correction image and the autofluorescence component correction image.
10. The information processing device according to clause 8, further including:
   an image generation unit that generates a spectral intensity ratio image representing a ratio between spectral intensity of each pixel in the fluorescence component correction image and spectral intensity of each pixel in the autofluorescence component correction image; and
   a storage unit that stores an imaging condition at a time of acquisition of the fluorescence image by imaging the specimen, and the spectral intensity ratio image, in association with each other.
11. The information processing device according to clause 1, further including a correction unit that corrects the fluorescence component image based on the fluorescence image, the fluorescence component image, and the autofluorescence component correction image,
   wherein the processing unit processes the corrected fluorescence component image based on the autofluorescence component correction image.
12. The information processing device according to clause 11,
   wherein the correction unit estimates features of the autofluorescent substance in the fluorescence image from the fluorescence component image and the autofluorescence component correction image, and corrects the fluorescence component image based on the estimated features.
13. The information processing device according to clause 12,
   wherein the correction unit estimates the features by using a trained model having the fluorescence component image and the autofluorescence component correction image as inputs.
14. The information processing device according to clause 13,
   wherein the trained model is a model trained by using unsupervised learning.
15. The information processing device according to clause 1, further including an acquisition unit that acquires the fluorescence image by imaging the specimen.
16. The information processing device according to clause 1,
   wherein the processing unit detects a specific cell included in the fluorescence image based on the processed fluorescence component image.
17. An information processing method including:
   separating a fluorescence image obtained by observing a specimen labeled with one or more fluorescent dyes into a fluorescence component image containing one or more fluorescence components and an autofluorescence component image containing one or more autofluorescence components;
   generating an autofluorescence component correction image using a reference spectrum of each of one or more autofluorescent substances included in the specimen and using the autofluorescence component image; and
   processing the fluorescence component image based on the autofluorescence component correction image.
18. A program for causing a computer to execute processes including:
   a process of separating a fluorescence image obtained by observing a specimen labeled with one or more fluorescent dyes into a fluorescence component image containing one or more fluorescence components and an autofluorescence component image containing one or more autofluorescence components;
   a process of generating an autofluorescence component correction image using a reference spectrum of each of one or more autofluorescent substances included in the specimen and using the autofluorescence component image; and
   a process of processing the fluorescence component image based on the autofluorescence component correction image.
19. A microscope system including:
   a light source that emits light to a specimen labeled with one or more fluorescent dyes; an imaging device that observes fluorescence emitted from the specimen irradiated with the light; and a program designed to execute processes on a fluorescence image acquired by the imaging device,
   wherein the program, by being run on a computer, causes the computer to execute processes including:
      a process of separating the fluorescence image into a fluorescence component image including one or more fluorescence components and an autofluorescence component image including one or more autofluorescence components;
      a process of generating an autofluorescence component correction image using a reference spectrum of each of one or more autofluorescent substances included in the specimen and the autofluorescence component image; and
      a process of processing the fluorescence component image based on the autofluorescence component correction image.
20. An analysis system including:
   the information processing device according to clause 1; and
   an analysis device that is connected to the information processing device via a predetermined network and analyzes the fluorescence component image processed by the information processing device.

## Claims

1. An information processing device including:
a separation unit configured to separate a fluorescence image obtained by observing a cell labeled with one or more fluorescent dyes into a fluorescence component image containing one or more fluorescence components and an autofluorescence component image containing one or more autofluorescence components;
a generation unit configured to generate an autofluorescence component correction image using a reference spectrum of each of one or more autofluorescent substances included in the cell and using the autofluorescence component image; and
a processing unit configured to generate a mask image using the autofluorescence component correction image; and
wherein the generation unit is configured to generate the autofluorescence component correction image by summing a result of multiplying the autofluorescence component image and the reference spectrum for each of the autofluorescent substances.

2. The information processing device according to claim 1, wherein the processing unit is configured to generate a fluorescence component correction image based on the mask image.

3. The information processing device according to claim 1, wherein the processing unit is configured to generate region information indicating a region labeled with the fluorescent dye from the autofluorescence component correction image, and to process the fluorescence component image using the generated region information.

4. The information processing device according to claim 3, wherein the region information is the mask image, and the mask image is a binary mask indicating a region labelled with the fluorescent dye.

5. The information processing device according to claim 4, wherein the processing unit is configured to generate the binary mask based on a magnitude relationship between a threshold set based on the autofluorescence component correction image and a pixel value of each pixel in the fluorescence image.

6. The information processing device according to claim 5, wherein the processing unit is configured to set a threshold for each of the pixels based on a pixel value of each of the pixels in the autofluorescence component correction image, and to generate the binary mask based on a magnitude relationship between the threshold for each of the pixels and a pixel value of each of the pixels in the fluorescence image.

7. The information processing device according to claim 1, further including an acquisition unit configured to acquire the fluorescence image by imaging the cell.

8. The information processing device according to claim 1, further including a correction unit configured to correct the fluorescence component image based on the fluorescence image, the fluorescence component image, and the autofluorescence component correction image, wherein the processing unit is configured to process the corrected fluorescence component image based on the autofluorescence component correction image.

9. The information processing device according to claim 8, wherein the correction unit is configured to estimate features of the autofluorescent substance in the fluorescence image from the fluorescence component image and the autofluorescence component correction image, and to correct the fluorescence component image based on the estimated features

10. The information processing device according to claim 9, wherein the correction unit is configured to estimate the features by using a trained model having the fluorescence component image and the autofluorescence component correction image as inputs.

11. The information processing device according to claim 10, wherein the trained model is a model trained by using unsupervised learning.

12. An information processing method including:
separating a fluorescence image obtained by observing a cell labeled with one or more fluorescent dyes into a fluorescence component image containing one or more fluorescence components and an autofluorescence component image containing one or more autofluorescence components;
generating an autofluorescence component correction image using a reference spectrum of each of one or more autofluorescent substances included in the cell and using the autofluorescence component image; and
generating a mask image using the autofluorescence component correction image;
wherein generating the autofluorescence component correction image comprises summing a result of multiplying the autofluorescence component image and the reference spectrum for each of the autofluorescent substances.

13. A program for causing a computer to execute processes including:
a process of separating a fluorescence image obtained by observing a cell labeled with one or more fluorescent dyes into a fluorescence component image containing one or more fluorescence components and an autofluorescence component image containing one or more autofluorescence components;
a process of generating an autofluorescence component correction image using a reference spectrum of each of one or more autofluorescent substances included in the cell and using the autofluorescence component image; and
a process of generating a mask image using the autofluorescence component correction image;
wherein the process of generating the autofluorescence component correction image comprises summing a result of multiplying the autofluorescence component image and the reference spectrum for each of the autofluorescent substances.

14. A microscope system including:
a light source configured to emit light to a cell labelled with one or more fluorescent dyes;
an imaging device configured to observe fluorescence emitted from the cell irradiated with the light; and
a program designed to execute processes on a fluorescence image acquired by the imaging device, wherein the program, by being run on a computer, causes the computer to execute processes including:
a process of separating a fluorescence image obtained by observing a cell labeled with one or more fluorescent dyes into a fluorescence component image containing one or more fluorescence components and an autofluorescence component image containing one or more autofluorescence components;
a process of generating an autofluorescence component correction image using a reference spectrum of each of one or more autofluorescent substances included in the cell and using the autofluorescence component image; and
a process of generating a mask image using the autofluorescence component correction image; and
wherein the process of generating the autofluorescence component correction image comprises summing a result of multiplying the autofluorescence component image and the reference spectrum for each of the autofluorescent substances.

15. An analysis system including:
the information processing device according to claim 1; and
an analysis device that is connected to the information processing device via a predetermined network and analyses the fluorescence component image generated by the information processing device.
